# EUROPEAN PATENT SPECIFICATION

(11) **EP 2 458 990 B1**
(45) Date of publication and mention of the grant of the patent: **30.03.2016**
(21) Application number: 10806872.7
(22) Date of filing: 27.07.2010
(51) Int. Cl.: A61K 38/00, A61K 9/19, A61K 47/00

(54) **METHODS FOR PRODUCING HIGH CONCENTRATION LYOPHILIZED PHARMACEUTICAL FORMULATIONS**
VERFAHREN ZUR HERSTELLUNG HOCHKONZENTRIERTER LYOPHILISIERTER PHARMAZEUTISCHER FORMULIERUNGEN
PROCÉDÉS DE PRODUCTION DE FORMULATIONS PHARMACEUTIQUES LYOPHILISÉES À CONCENTRATION ÉLEVÉE

(30) Priority: 28.07.2009 US 229141 P
(43) Date of publication of application: 06.06.2012
(73) Proprietor: Merck Sharp & Dohme Corp., Rahway, NJ 07065-0907 (US)
(72) Inventor: BHAMBHANI, Akhilesh, Rahway New Jersey 07065-0907 (US); MEYER, Brian, K., Rahway New Jersey 07065-0907 (US); BLUE, Jeffrey, T., Rahway New Jersey 07065-0907 (US)
(74) Representative: Böhles, Elena
(86) International application number: PCT/US2010/043313
(87) International publication number: WO 2011/017070

(56) References cited:
- US-A1- 2004 191 243
- US-A1- 2004 191 243
- US-A1- 2008 213 215
- US-A1- 2008 213 215

### SEQUENCE LISTING

<110> Merck Sharp & Dohme Corp.
<120> METHODS FOR PRODUCING HIGH CONCENTRATION LYOPHILIZED PHARMACEUTICAL FORMULATIONS
<130> MRL-MIS-00015
<150> 61/229,141
   <151> 2009-07-28
<160> 8
<170> FastSEQ for Windows Version 4.0
<210> 1
   <211> 118
   <212> PRT
   <213> Artificial Sequence
<220>
   <223> 10G5-6 VH
<400> 1
   Glu Val Gln Leu Val Gln Ser Gly Ala Glu Val Lys Lys Pro Gly Glu
   1 5 10 15
   Ser Leu Lys Ile Ser Cys Lys Gly Ser Gly Tyr Ser Phe Thr Ser Tyr
   20 25 30
   Trp Ile Gly Trp Val Arg Gln Met Pro Gly Lys Gly Leu Glu Trp Met
   35 40 45
   Gly Val Ile Tyr Pro Gly Asp Ser Tyr Thr Arg Tyr Ser Pro Ser Phe
   50 55 60
   Gln Gly Gln Val Thr Ile Ser Ala Asp Lys Ser Ile Ser Thr Ala Tyr
   65 70 75 80
   Leu Gln Trp Ser Ser Leu Lys Ala Ser Asp Thr Ala Met Tyr Tyr Cys
   85 90 95
   Ala Arg Met Pro Asn Trp Gly Ser Leu Asp His Trp Gly Gln Gly Thr
   100 105 110
   Leu Val Thr Val Ser Ser
   115
<210> 2
   <211> 104
   <212> PRT
   <213> Artificial Sequence
<220>
   <223> 10G5-6 VL
<400> 2
   Glu Ile Val Leu Thr Gln Ser Pro Gly Thr Leu Ser Leu Ser Pro Gly
   1 5 10 15
   Glu Arg Ala Thr Leu Ser Cys Arg Ala Ser Gln Ser Ile Ser Ser Ser
   20 25 30
   Tyr Leu Ala Trp Tyr Gln Gln Lys Pro Gly Gln Ala Pro Arg Leu Leu
   35 40 45
   Ile Tyr Gly Ala Ser Ser Arg Ala Thr Gly Ile Pro Asp Arg Phe Ser
   50 55 60
   Gly Ser Gly Ser Gly Thr Asp Phe Thr Leu Thr Ile Ser Arg Leu Glu
   65 70 75 80
   Pro Glu Asp Phe Ala Val Tyr Tyr Cys Gln Gln Tyr Ala Ser Phe Gly
   85 90 95
   Gln Gly Thr Lys Val Glu Ile Lys
   100
<210> 3
   <211> 438
   <212> PRT
   <213> Artificial Sequence
<220>
   <223> Rh2-18 antibody heavy chain without leaders
<400> 3
   Glu Val Gln Leu Val Gln Ser Gly Ala Glu Val Lys Lys Pro Gly Ala
   1 5 10 15
   Ser Val Lys Val Ser Cys Lys Ala Ser Gly Tyr Thr Phe Thr Asp Tyr
   20 25 30
   Tyr Ile His Trp Val Arg Gln Ala Pro Gly Gln Gly Leu Glu Trp Met
   35 40 45
   Gly Trp Ile His Ser Asn Ser Gly Ala Thr Thr Tyr Ala Gln Lys Phe
   50 55 60
   Gln Ala Arg Val Thr Met Ser Arg Asp Thr Ser Ser Ser Thr Ala Tyr
   65 70 75 80
   Met Glu Leu Ser Arg Leu Glu Ser Asp Asp Thr Ala Met Tyr Phe Cys
   85 90 95
   Ser Arg Glu Asp Tyr Trp Gly Gln Gly Thr Leu Val Thr Val Ser Ser
   100 105 110
   Ala Ser Thr Lys Gly Pro Ser Val Phe Pro Leu Ala Pro Cys Ser Arg
   115 120 125
   Ser Thr Ser Glu Ser Thr Ala Ala Leu Gly Cys Leu Val Lys Asp Tyr
   130 135 140
   Phe Pro Glu Pro Val Thr Val Ser Trp Asn Ser Gly Ala Leu Thr Ser
   145 150 155 160
   Gly Val His Thr Phe Pro Ala Val Leu Gln Ser Ser Gly Leu Tyr Ser
   165 170 175
   Leu Ser Ser Val Val Thr Val Thr Ser Ser Asn Phe Gly Thr Gln Thr
   180 185 190
   Tyr Thr Cys Asn Val Asp His Lys Pro Ser Asn Thr Lys Val Asp Lys
   195 200 205
   Thr Val Glu Arg Lys Cys Cys Val Glu Cys Pro Pro Cys Pro Ala Pro
   210 215 220
   Pro Val Ala Gly Pro Ser Val Phe Leu Phe Pro Pro Lys Pro Lys Asp
   225 230 235 240
   Thr Leu Met Ile Ser Arg Thr Pro Glu Val Thr Cys Val Val Val Asp
   245 250 255
   Val Ser Gln Glu Asp Pro Glu Val Gln Phe Asn Trp Tyr Val Asp Gly
   260 265 270
   Val Glu Val His Asn Ala Lys Thr Lys Pro Arg Glu Glu Gln Phe Asn
   275 280 285
   Ser Thr Phe Arg Val Val Ser Val Leu Thr Val Leu His Gln Asp Trp
   290 295 300
   Leu Asn Gly Lys Glu Tyr Lys Cys Lys Val Ser Asn Lys Gly Leu Pro
   305 310 315 320
   Ser Ser Ile Glu Lys Thr Ile Ser Lys Thr Lys Gly Gln Pro Arg Glu
   325 330 335
   Pro Gln Val Tyr Thr Leu Pro Pro Ser Arg Glu Glu Met Thr Lys Asn
   340 345 350
   Gln Val Ser Leu Thr Cys Leu Val Lys Gly Phe Tyr Pro Ser Asp Ile
   355 360 365
   Ala Val Glu Trp Glu Ser Asn Gly Gln Pro Glu Asn Asn Tyr Lys Thr
   370 375 380
   Thr Pro Pro Met Leu Asp Ser Asp Gly Ser Phe Phe Leu Tyr Ser Lys
   385 390 395 400
   Leu Thr Val Asp Lys Ser Arg Trp Gln Gln Gly Asn Val Phe Ser Cys
   405 410 415
   Ser Val Met His Glu Ala Leu His Asn His Tyr Thr Gln Lys Ser Leu
   420 425 430
   Ser Leu Ser Pro Gly Lys
   435
<210> 4
   <211> 217
   <212> PRT
   <213> Artificial Sequence
<220>
   <223> RH2-18 antibody light chain without leaders
<400> 4
   Gln Ser Val Leu Thr Gln Pro Pro Ser Val Ser Gly Ala Pro Gly Gln
   1               5                   10                  15
   Arg Val Thr Ile Ser Cys Thr Gly Ser Ser Ser Asn Ile Gly Ala Gly
               20                  25                  30
   Tyr Asp Val His Trp Tyr Gln Gln Leu Pro Gly Thr Ala Pro Lys Leu
           35                  40                  45
   Leu Ile Tyr Gly Tyr Ser Asn Arg Pro Ser Gly Val Pro Asp Arg Phe
       50                  55                  60
   Ser Gly Ser Lys Ser Gly Ala Ser Ala Ser Leu Ala Ile Thr Gly Leu
   65                  70                  75                  80
   Arg Pro Asp Asp Glu Ala Asp Tyr Tyr Cys Gln Ser Tyr Asp Asn Ser
                   85                  90                  95
   Leu Ser Ser Tyr Val Phe Gly Gly Gly Thr Gln Leu Thr Val Leu Ser
               100                 105                 110
   Gln Pro Lys Ala Asn Pro Thr Val Thr Leu Phe Pro Pro Ser Ser Glu
           115                 120                 125
   Glu Leu Gln Ala Asn Lys Ala Thr Leu Val Cys Leu Ile Ser Asp Phe
       130                 135                 140
   Tyr Pro Gly Ala Val Thr Val Ala Trp Lys Ala Asp Gly Ser Pro Val
   145                 150                 155                 160
   Lys Ala Gly Val Glu Thr Thr Lys Pro Ser Lys Gln Ser Asn Asn Lys
                   165                 170                 175
   Tyr Ala Ala Ser Ser Tyr Leu Ser Leu Thr Pro Glu Gln Trp Lys Ser
               180                 185                 190
   His Arg Ser Tyr Ser Cys Gln Val Thr His Glu Gly Ser Thr Val Glu
           195                 200                 205
   Lys Thr Val Ala Pro Thr Glu Cys Ser
       210                 215
<210> 5
   <211> 123
   <212> PRT
   <213> Artificial Sequence
<220>
   <223> hu20c2a3 VH
<400> 5
   Gln Val Thr Leu Lys Glu Ser Gly Pro Gly Leu Leu Lys Pro Thr Gln
   1               5                   10                  15
   Thr Leu Thr Leu Thr Cys Thr Leu Ser Gly Phe Ser Leu Ser Thr Ser
               20                  25                  30
   Gly Met Gly Val Gly Trp Phe Arg Gln Pro Pro Gly Lys Gly Leu Glu
           35                  40                  45
   Trp Leu Ala His Ile Trp Trp Asp Asp Asp Lys Ser Tyr Asn Pro Ser
       50                  55                  60
   Leu Lys Ser Arg Leu Thr Ile Ser Lys Asp Thr Ser Lys Asn Gln Val
   65                  70                  75                  80
   Val Leu Thr Ile Thr Asn Val Asp Pro Val Asp Thr Ala Thr Tyr Tyr
                   85                  90                  95
   Cys Ala Arg Arg Gln Leu Gly Thr Arg Gly Thr Asp Ala Met Asp Tyr
               100                 105                 110
   Trp Gly Gln Gly Thr Thr Val Thr Val Ser Ser
           115                 120
<210> 6
   <211> 112
   <212> PRT
   <213> Artificial Sequence
<220>
   <223> hu20c2a3 VL
<400> 6
   Asp Val Val Met Thr Gln Thr Pro Leu Ser Leu Pro Val Thr Pro Gly
   1               5                   10                  15
   Gln Pro Ala Ser Ile Ser Cys Arg Ser Ser Gln Ser Ile Leu His Ser
               20                  25                  30
   Asn Gly Asn Thr Tyr Leu Glu Trp Tyr Leu Gln Lys Pro Gly Gln Ser
           35                  40                  45
   Pro Gln Leu Leu Ile Tyr Lys Val Ser Asn Arg Phe Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Leu Gln Thr
                   85                  90                  95
   Thr Arg Val Pro Leu Thr Phe Gly Gln Gly Thr Lys Leu Glu Ile Lys
               100                 105                 110
<210> 7
   <211> 123
   <212> PRT
   <213> Artificial Sequence
<220>
   <223> 20c2 humanized VH
<400> 7
   Gln Val Thr Leu Lys Glu Ser Gly Pro Ala Leu Val Lys Pro Thr Gln
   1               5                   10                  15
   Thr Leu Thr Leu Thr Cys Thr Leu Ser Gly Phe Ser Leu Ser Thr Ser
               20                  25                  30
   Gly Met Gly Val Gly Trp Ile Arg Gln Pro Pro Gly Lys Ala Leu Glu
           35                  40                  45
   Trp Leu Ala His Ile Trp Trp Asp Asp Asp Lys Ser Tyr Asn Pro Ser
       50                  55                  60
   Leu Lys Ser Arg Leu Thr Ile Ser Lys Asp Thr Ser Lys Asn Gln Val
   65                  70                  75                  80
   Val Leu Thr Met Thr Asn Met Asp Pro Val Asp Thr Ala Thr Tyr Tyr
                   85                  90                  95
   Cys Ala Arg Arg Gln Leu Gly Leu Arg Ser Ile Asp Ala Met Asp Tyr
               100                 105                 110
   Trp Gly Gln Gly Thr Thr Val Thr Val Ser Ser
           115                 120
<210> 8
   <211> 114
   <212> PRT
   <213> Artificial Sequence
<220>
   <223> 20c2 humanized VL
<400> 8
   Asp Val Val Met Thr Gln Ser Pro Leu Ser Leu Pro Val Thr Pro Gly
    1               5                  10                  15
   Glu Pro Ala Ser Ile Ser Cys Arg Ser Ser Gln Ser Ile Leu His Ser
               20                  25                  30
   Asn Gly Asn Thr Tyr Leu Glu Trp Tyr Leu Gln Lys Pro Gly Gln Ser
           35                  40                  45
   Pro Gln Leu Leu Ile Tyr Lys Val Ser Asn Arg Phe Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Phe Gln Gly
                   85                  90                  95
   Ser Leu Val Pro Leu Thr Phe Gly Gln Gly Thr Lys Leu Glu Ile Lys
               100                 105                 110
   Arg Thr

## Description

### FIELD OF THE INVENTION

The invention relates to stable, lyophilized pharmaceutical formulations comprising a high concentration of protein or antibody and methods of producing such formulations.

### BACKGROUND OF THE INVENTION

The successful use of low potency antibodies and other low potency therapeutic proteins in a biological product is dependent on the development of a stable formulation comprising the therapeutic protein and/or antibody at a high concentration. This is particularly true if the desired mode of administration of the product is subcutaneous, as this mode of delivery requires small volumes of product (∼1-1.5 ml). High concentration protein or antibody formulations may also be required when using a frequent dosing regimen with high doses (several mg/kg) for an intravenous route (IV) or intramuscular route (IM) of administration. Although liquid formulations can generally be manufactured with a less complex, shorter manufacturing process than a comparable lyophilized formulation, they are typically less stable and prone to physical degradation, leading to a loss of active product during the storage and distribution period. For this reason, development of a lyophilized product is often the preferred method of attaining a stable, high concentration formulation that is suitable for the contemplated mode of administration, including subcutaneous.

Historically, high concentration lyophilized formulations were obtained by lyophilizing a formulation comprising a lower protein concentration (∼ 20-50 mg/ml), relative to the high concentration desired for the final reconstituted product (e.g. 70-250 mg/ml) and reconstituting the resulting lyophilized cake to 2-40 times greater concentration, using a smaller volume of diluent. *See, e.g.* Liu et al., U.S. Patent 6,875,432, and Andya et al., U.S. Patent 6,267,958. While formulations comprising a high concentration of protein or antibody could be achieved using this method, there are significant disadvantages to using this method, including a less efficient process as the concentration of the drug product is different than the drug substance. Additionally, since achieving a highly concentrated reconstituted formulation in the desired volume would require a larger volume of lower-concentration product prior to lyophilization, use of this method requires larger vials and a greater amount of bulk storage space, process space, and shipping and handling requirements than methods relying on high concentrations of protein prior to lyophilization, resulting in a higher cost of goods.

Methods of obtaining high concentration lyophilized formulations that utilize a high concentration of protein or antibody prior to lyophilization are known, but these methods require a lengthy reconstitution time post-lyophilization and are not suitable for subcutaneous injection. Such lengthy reconstitution times are not optimal for lyophilized pharmaceutical formulations prepared and administered at home. A high-concentration, lyophilized formulation with a quick reconstitution time would also be more efficient and advantageous for reconstituted liquid formulations prepared by healthcare workers or pharmacists.

Two distinct phases that are necessary for the successful development of a lyophilized formulation, namely, optimization of the formulation itself and optimization of the lyophilization cycle, are identified in the prior art. (*See, e.g.,* Carpenter et al., Rational Design of Stable Lyophilized Protein Formulations: Some Practical Advice. Pharm Res. 14: 969-975 (1997); Sarciaux et al., Effects of Buffer Conditions on Aggregation of Bovine IgG during Freeze-Drying. J. Pharm. Sci. 12: 1354-1361 (1999); Chang et al. Surface-Induced Denaturation of Proteins during Freezing and its Inhibition by Surfactants. J. Pharm. Sci. 12:1325-1330 (1996)). Publications focusing on formulation design include Barry et al. (WO 2008/086395) and Goldstein et al. (WO 2007/095337), which disclose lyophilized formulations comprising an antibody, a cryoprotectant, and a buffer; and Schulke et al. (US 2005/0142139), which discloses formulations comprising a high concentration of CD4-IgG2 antibodies and a histidine buffer. Publications describing liquid formulations comprising a high concentration of antibody are also known. *See, e.g.,* Kaisheva et al., US 2003/0138417 and Liu et al., US 2007/0053900.

Although a significant number of publications have disclosed optimization of the lyophilization cycle, most of these reports focus on the reduction of primary drying time with fewer reports focusing on the reduction of the reconstitution time of high concentration lyophilized protein formulations. (*See, e.g.*, Harris et al., Commercial Manufacturing Scale Formulation and Analytical Characterization of Therapeutic Recombinant Antibodies. Drug Dev. Res. 61: 137-154 (2004); Shire, et al., Challenges in Development of High Protein Concentration Formulations. J. Pharm. Sci. 6: 1390-1402 (2004); Blue et al., Successful Lyophilization Development of Protein Therapeutics. Am. Pharm. Rev. 40-44 (2009)). For example, Colandene et al. (WO 2006/081320) disclose a lyophilization method in which the temperature of the primary drying step is above the glass transition temperature (Tg') of the formulation, but below the collapse temperature (Tc). However, Collandene *et al.* did not discuss reconstitution time post-lyophilization.

Blue *et al.* (*supra*) recently reported that increasing the protein concentration of the formulation prior to lyophilization allows for the development of a more aggressive primary drying time; however, the reconstitution time reported is longer than one hour at high protein concentrations (68 minutes for 100 mg/ml mAb). Blue *et al.* (*supra*) also reported that the addition of an annealing step to the lyophilization cycle reduced the reconstitution time of the lyophilized formulation to 47 minutes relative to a comparable non-annealed sample, which required 68 minutes to reconstitute. Although the addition of an annealing step can shorten the reconstitution time, the speed of reconstitution of annealed formulations under the reported conditions was not sufficient for high dose, chronic usage formulations for at-home administration.

Kaisheva et al. (WO 2003/009817) disclose lyophilized antibody compositions comprising 50 mg/ml or more of an IgG antibody, histidine buffer, polysorbate, and sucrose, which are stated to reconstitute in less than 2 minutes. However, reconstitution times are only provided for formulations comprising 50 mg/ml antibody or less. Similarly, Barry *et al.* (*supra*) disclose formulations comprising 50 mg/ml antibody, a cryoprotectant, and a buffer, which reconstituted in less than 3 minutes. However, similar to other prior art methods, Barry *et al.* used a higher fill volume of a lower concentration formulation (50 mg/ml) prior to lyophilization and reconstituted with a smaller volume to achieve a high concentration reconstituted formulation.

Therefore, development of stable, lyophilized formulations comprising a high concentration of the desired active biological ingredient with a fast reconstitution time, and methods of producing such high concentration formulations are desirable and would fulfill an unmet need.

### SUMMARY OF THE INVENTION

The present invention relates, in part, to methods of producing lyophilized pharmaceutical compositions comprising a high concentration of therapeutic protein or antibody prior to lyophilization, wherein the lyophilized formulation can be reconstituted with a diluent in a short period of time, preferably about 15 minutes or less. The invention also relates to the high concentration lyophilized formulations produced by the methods described herein. The lyophilized formulations produced by the methods of the invention are stable and are suitable for veterinary and human medical use and are suitable for modes of administration including oral, pulmonary and parenteral, such as intravenous, intramuscular, intraperitoneal, or subcutaneous injection.

In some embodiments of the methods described herein, the liquid formulations prior to lyophilization comprise an active biological/pharmaceutical therapeutic agent, which is either an antibody present at a concentration between about 70 mg/ml and 250 mg/ml or a therapeutic protein present at a concentration of about 5 mg/ml to about 60 mg/ml. The liquid formulations prepared for use in the freeze-drying methods of the invention also comprise a bulking agent and in some cases, a stabilizer or solubilizer, and may also comprise other excipients such as a buffer, or a surfactant. The lyophilized formulation is stable for at least 1 month at room temperature, and preferably at least 6 months.

The present invention also provides a pharmaceutical composition comprising (i) about 70-250 mg/ml of antibody or about 5 mg/ml to about 60 mg/ml of therapeutic protein or peptide, (ii) about 1% to about 6% w/v sucrose or trehalose, (iii)about 25 mM to about 100 mM histidine, succinate or bis-tris, (iv) about 25 mM to about 100 mM arginine and (v) about 1% to about 8% w/v mannitol, wherein the pH of the formulation is about 5.5 to about 7.5. The pharmaceutical composition may optionally comprise a nonionic surfactant, including, but not limited to polysorbate 80 or polysorbate 20.

As used throughout the specification and in the appended claims, the singular forms "a," "an," and "the" include the plural reference unless the context clearly dictates otherwise.

As used throughout the specification and appended claims, the following definitions and abbreviations apply:
The term "SWFI" refers to sterile water for injection.
The term "BWFI" refers to bacteriostatic water for injection, which is sterile water comprising an antimicrobial preservative.
"Tg" stands for the glass transition temperature of a lyophilized product, which is the temperature at which the amorphous components of an amorphous solution changes from a viscous liquid to a glass. The glass transistion temperature of a frozen solution prior to drying is referred to as "Tg'."
"Teu" stands for "eutectic temperature," which is the temperature at which all of the constituents of a crystalline solution (i.e. solute and solvent) become crystalline or frozen simultaneously.
"Tc" stands for the collapse temperature of a product, which is the temperature at which disappearance of freezing pattern is observed at the sublimation interface of the lyophilization matrix during the process of freeze drying.
"Shelf set point temperature" refers to the temperature of the shelf inside the lyophilizer on which the containers or vials are set during the process of lyophilization.
The term "API" refers to an active pharmaceutical ingredient, which is a component of the formulations disclosed herein that is pharmaceutically or biologically active and confers a therapeutic or prophylactic benefit to a person or animal in need thereof. As used herein, an API can be an antibody or fragment thereof, a therapeutic protein, a therapeutic peptide, or a vaccine active ingredient. API is used interchangeably herein with the term "active biological ingredient."
"Formulation" refers to a composition containing an active pharmaceutical or biological ingredient, along with one or more addition components. The formulations can be liquid or solid (e.g., lyopholized). Additional components that may be included as appropriate include pharmaceutically acceptable excipients, additives, diluents, buffers, sugars, amino acids (such as glycine, glutamine, asparagine, arginine or lysine), chelating agents, surfactants, polyols, bulking agents, stabilizers, lyoprotectants, solubilizers, emulsifiers, salts, adjuvants, tonicity enhancing agents (such as alkali metal halides, preferably sodium or potassium chloride, mannitol, sorbitol), delivery vehicles and anti-microbial preservatives.

Acceptable formulation components for pharmaceutical/biological preparations are nontoxic to recipients at the dosages and concentrations employed. Typically, the "formulation" is a single dose of active pharmaceutical ingredient ("API"), which can be delivered to a single patient or animal in need thereof. However, the term "multi-dose" refers to a formulation which contains more than one dose of an API which can be administered to a patient more than one time. A multi-dose formulation typically comprises an anti-microbial preservative. The term "formulation" is used interchangeably herein with the terms "composition," "biological composition," and "pharmaceutical composition."

The term "cake" refers to a dry pellet that results when a liquid formulation has been lyophilized or freeze-dried, as described herein. The appearance of the cake is partially indicative of the impact of the lyophilization process on the properties of the lyophilized formulation. As used herein, "dry cake" refers to a cake that comprises about 20% or less residual moisture content. In some embodiments of the invention, the moisture content of the dry cake is 15% or less, 10% or less, or 5% or less. In alternative embodiments, the moisture content of the dry cake is within a range of about 0.1% to about 10%, about 0.1% to about 6%, about 0.5% to about 10% or 0.5% to about 6%.

The term "bulking agent" means a substance or component, such as mannitol, lactose, disaccharide or glycine, that adds mass to a lyophilized formulation. A bulking agent may contribute to the physical structure, uniformity, and stability of the lyophilized cake.

The term "3/50/50 buffer," used interchangeably with "3/50/50," refers to a buffer comprising 3% sucrose, 50 mM histidine, and 50 mM arginine. The 3/50/50 buffer has a pH of about 6.0.

The term "1/50/50 buffer," used interchangeably with "1/50/50," refers to a buffer composition comprising 1% sucrose, 50 mM histidine, and 50 mM arginine. The 1/50/50 buffer has a pH of about 6.0.

The term "6/100/100 buffer," used interchangeably with "6/100/100," refers to a buffer composition comprising 6% sucrose, 100 mM histidine, and 100 mM arginine. The 6/100/100 buffer has a pH of about 6.0.

The term "reconstitution time" refers to the time that is required to rehydrate a dry, lyophilized, formulation (cake) so that the resulting reconstituted liquid formulation is clarified and the cake has been dissolved.

The term "therapeutically effective amount" refers to an amount of the active ingredient (i.e. therapeutic protein or antibody) sufficient to produce the desired therapeutic effect in a human or animal, e.g. the amount necessary to treat, cure, prevent, or inhibit development and progression of disease or the symptoms thereof and/or the amount necessary to ameliorate symptoms or cause regression of disease. Such a therapeutically effective amount may vary depending on the structure and potency of the active ingredient and the contemplated mode of administration. One of skill in the art can readily determine a therapeutically effective amount of a given antibody or protein.

The term "therapeutic protein" or "therapeutic peptide" refers to a biological protein or peptide, respectively, which is useful in the treatment of a disease or condition.

The term "treatment" refers to both therapeutic treatment and prophylactic or preventative measures. Those in need of treatment include those individuals, such as humans and animals, already with the disorder or condition to be treated as well as those prone to have the disorder or those in which the disorder is to be prevented. As used herein, "treatment" also includes reduction of the likelihood of obtaining the disorder, reduction of the severity of the disorder in those already afflicted, and the induction of regression of the disorder or symptoms thereof.

Additional abbreviations used herein include the following: ANS= 8-anilino-1-naphthalenesulfonate dye; API= active pharmaceutical ingredient; Arg= arginine; form. = formulation; His=histidine; hr. = hour(s); lyo= lyophilization; Mann= mannitol; min.= minute(s); PS20= polysorbate 20; PS80= polysorbate 80; recon.= reconstitution; s.= second(s), SC= subcutaneous; SDS= sodium dodecyl sulfate; SDS-PAGE= SDS polyacrylamide gel electrophoresis; SEC= size exclusion chromatography; vol.= volume; w/v= weight per volume.

### BRIEF DESCRIPTION OF THE DRAWINGS

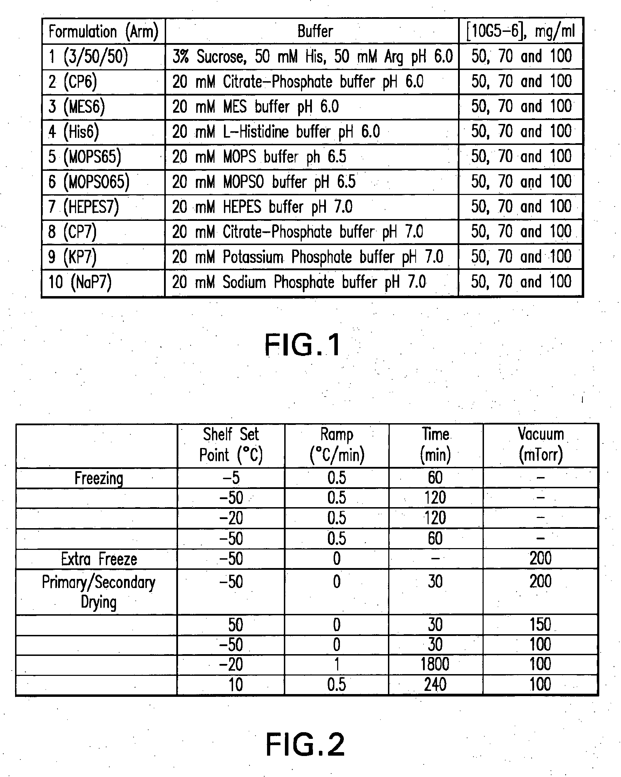

FIGURE 1 shows the buffers used for the initial lyophilization screen (Example 1). Concentrated stock solution of 10G5-6 at >100 mg/ml was dialyzed against the buffers to obtain the desired formulation. All formulations comprised 3% sucrose. Samples were lyophilized at a starting volume of 1 mL and starting concentration of 10G5-6 of approximately 50 mg/ml, 70 mg/ml and 100 mg/ml.
FIGURE 2 shows the lyophilization cycle parameters that were used in the initial formulation screen, as described in Example 1.

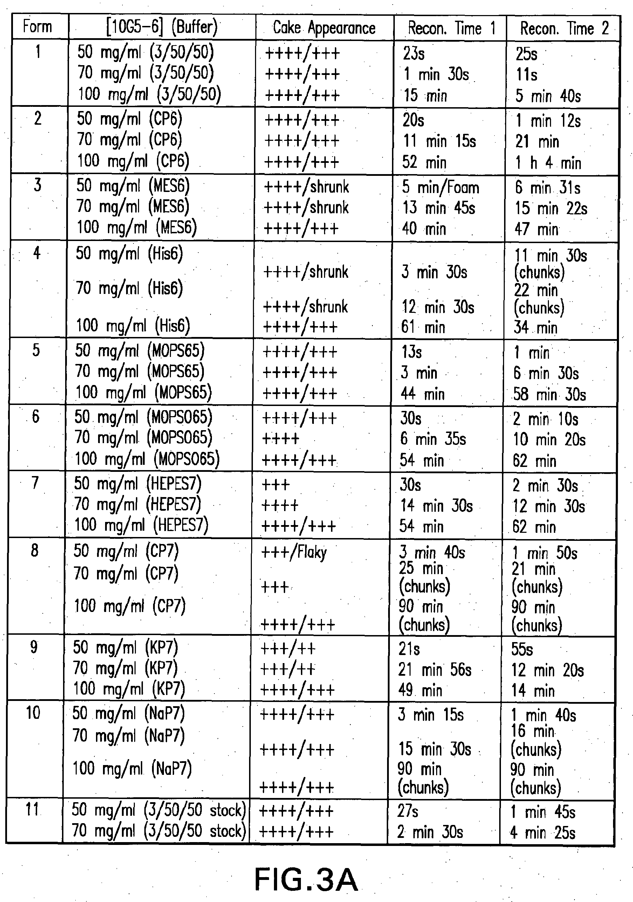

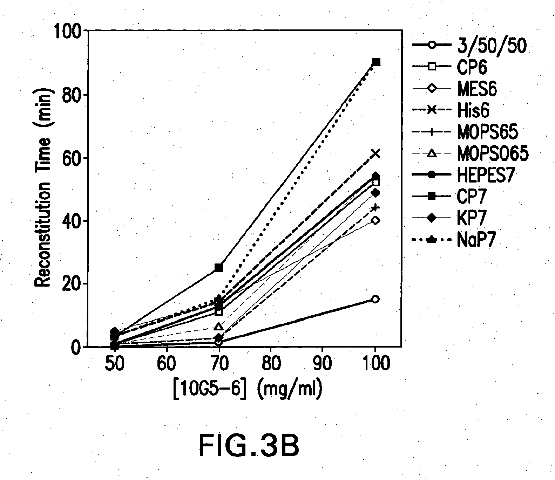

FIGURE 3 shows the reconstitution time and cake appearance of three formulations comprising different concentrations of 10G5-6, as described in Example 1 (panel A). The vials were reconstituted with 1 ml SWFI (Recon Time1) or with 1 ml SWFI containing 0.01% polysorbate 20 (PS20; Recon Time 2). The following symbols were used to denote the physical appearance of the cake: (1) ++++ elegant cake, no cracking; (2) +++ less intact, slight cracking; (3) ++ collapsed cake, more cracking; and (4) + completely collapsed cake. Also shown is a graph illustrating that the reconstitution times of the tested formulations reconstituted with SWFI increased with increasing protein concentration (panel B).

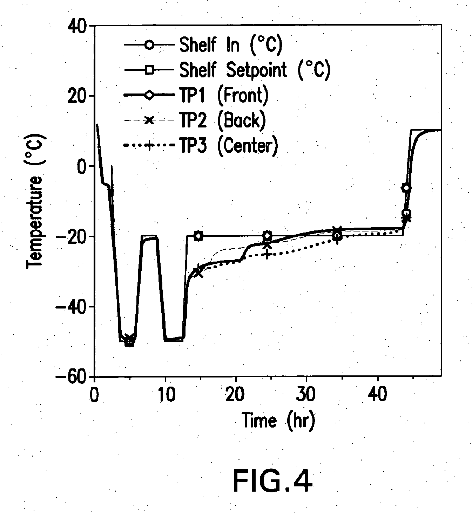

FIGURE 4 shows the product temperature profile during lyophilization of the initial formulations (Example 1) in the front (TP1), back (TP2) and center (TP3) of the lyophilization tray. Also shown is the temperature of the shelf and the shelf set point temperature (°C).

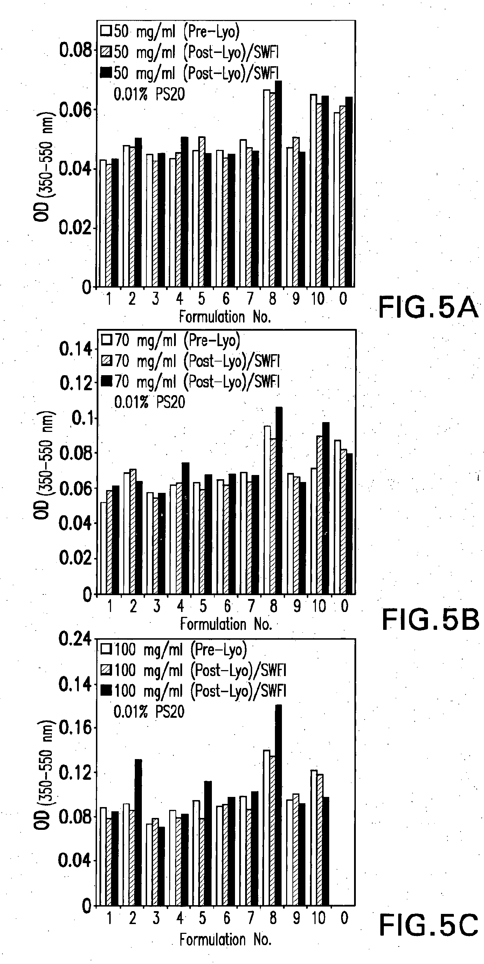

FIGURE 5 shows the results of the turbidity measurements used to determine whether the lyophilization and reconstitution process had an impact on the stability of 10G5-6 at 50 mg/ml (panel A), 70 mg/ml (panel B) and 100 mg/ml (panel C) concentration in the initial test formulations (Example 1). The results are shown for each formulation (as numbered in FIGURE 3) pre-lyophilization (clear bars), and post-lyophilization, reconstituted with SWFI (hatched bars) or SWFI + 0.01% PS20 (black bars). Form #0 corresponds to the stock 10G5-6 solution in 3/50/50 buffer.

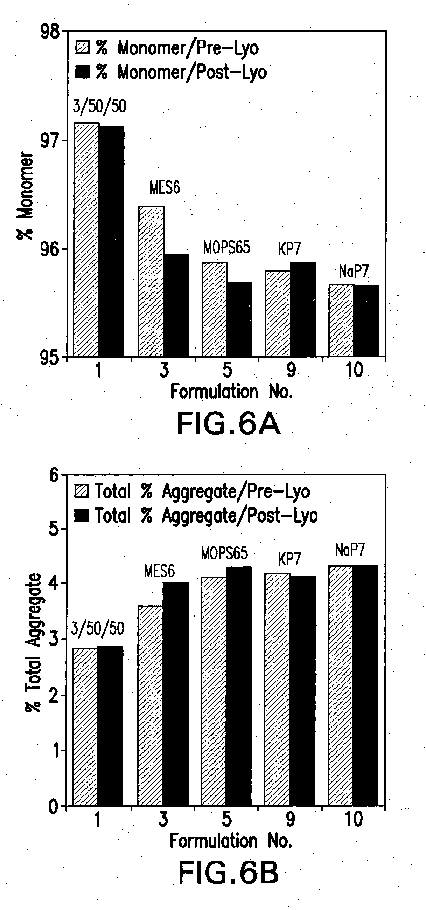

FIGURE 6 shows the percentage of monomers (Panel A) and total aggregation (Panel B) in the indicated formulations of 10G5-6 at 100 mg/ml concentration (see Example 1), as measured by SEC-HPLC, before and after lyophilization.

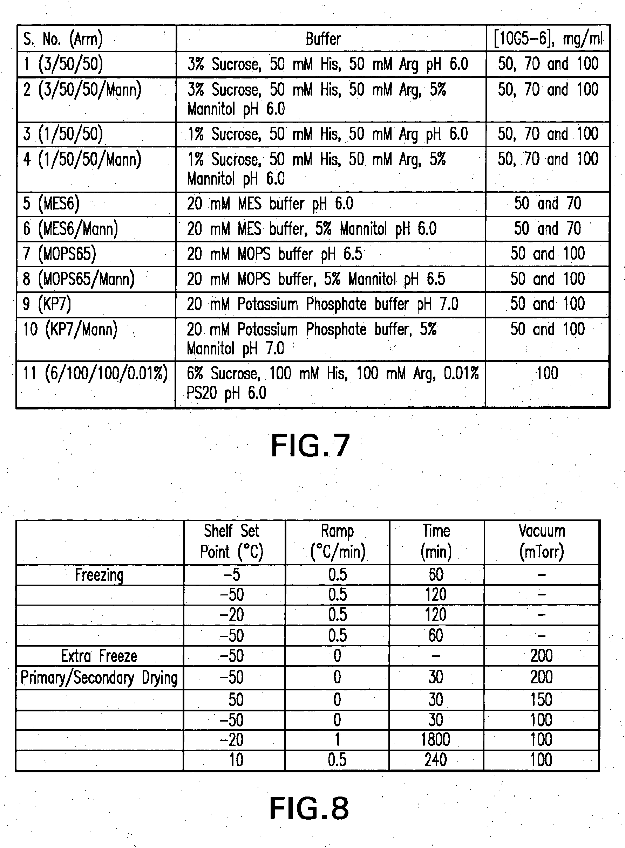

FIGURE 7 shows the buffers tested in the secondary lyophilization screen (Example 2). Concentrated stock solution of 10G5-6 at >100 mg/ml was dialyzed against the buffers to obtain the desired formulation. Sucrose (1 % and 3%) was added to some formulations (#'s 5-10) post-dialysis. Samples were lyophilized at a starting volume of 1 mL and starting concentration of approximately 50 mg/ml, 70 mg/ml and 100 mg/ml.
FIGURE 8 shows the lyophilization cycle parameters used for the secondary screen, as described in Example 2.

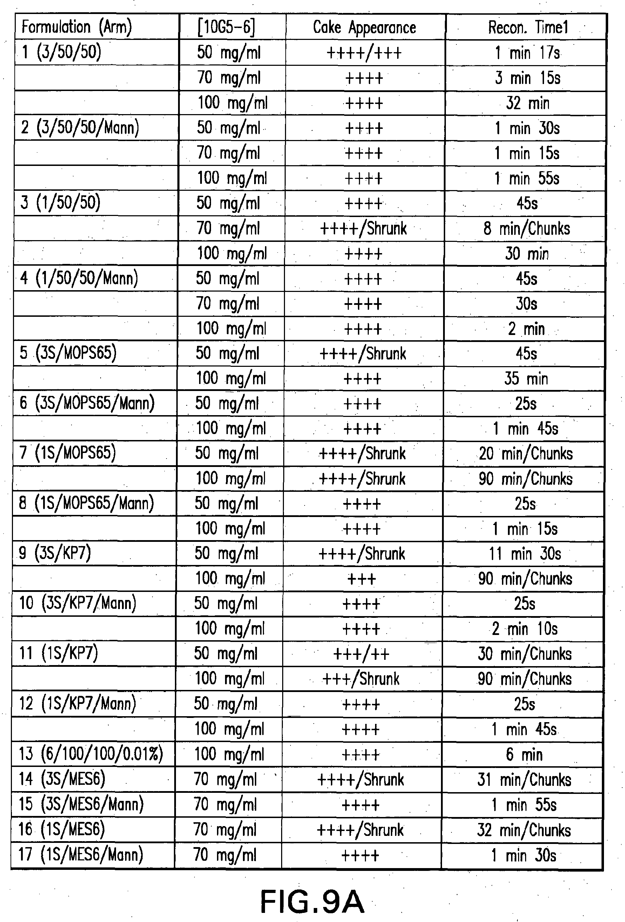

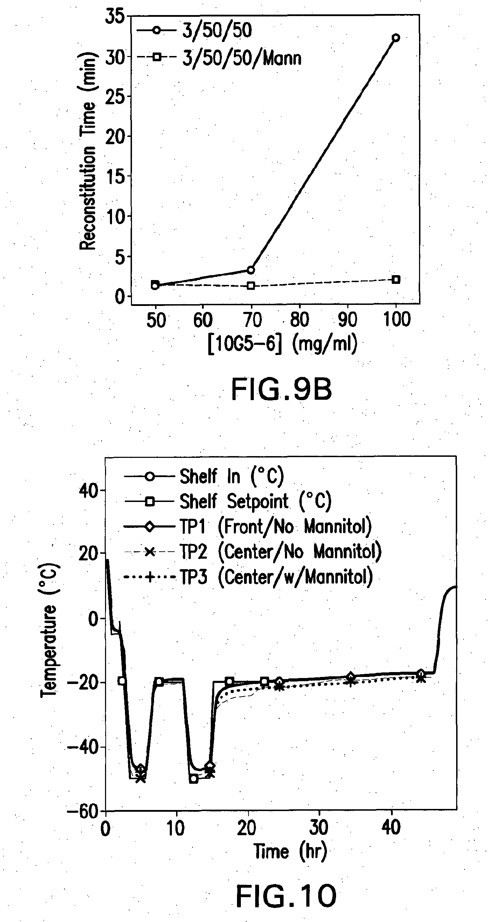

FIGURE 9 shows the reconstitution time and cake appearance of the lyophilized formulations described in Example 2 at three different concentrations of 10G5-6. The vials were reconstituted with 1 ml SWFI (Recon. Time 1). The following symbols were used to denote the appearance of the cake: (1) ++++ elegant cake, no cracking; (2) +++ less intact, slight cracking; (3) ++ collapsed cake, more cracking; and (4) + completely collapsed cake. Also shown is a graph of the reconstitution times for formulations comprising 3/50/50 and 1/50/50 buffers, with and without mannitol, reconstituted with SWFI (panel B).
FIGURE 10 shows the product temperature profile of the formulations tested in the secondary screen (Example 2) during lyophilization in the front (TP1), and center (TP2 (samples without mannitol) and TP3 (samples with mannitol)) of the lyophilization tray. Also shown is the temperature of the shelf and the shelf set point temperature (°C).

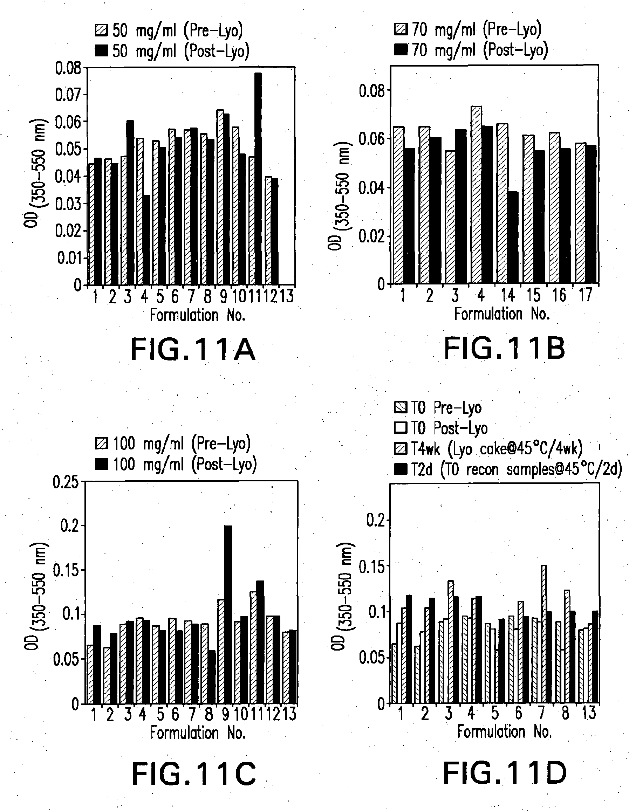

FIGURE 11 shows the turbidity measurements of the 10G5-6 formulations in the secondary screen (Example 2, see also FIGURE 9), which were obtained by measuring the OD350 to determine whether the lyophilization and reconstitution process had an impact on the stability of the formulations at 50 mg/ml (panel A), 70 mg/ml (panel B), and 100 mg/ml (panel C) antibody concentration. The results are shown for each formulation pre-lyophilization (hatched bars) and post-lyophilization (black bars). For comparison purposes, the turbidity measurements of the T0 reconstituted samples (pre- and post-lyophilization) comprising 100mg/ml antibody, as well as the lyophilized cakes stored at 45°C for 4 weeks (T4wk) and the T0 reconstituted samples at 45° C for 2 days (T2d) are also shown (panel D).

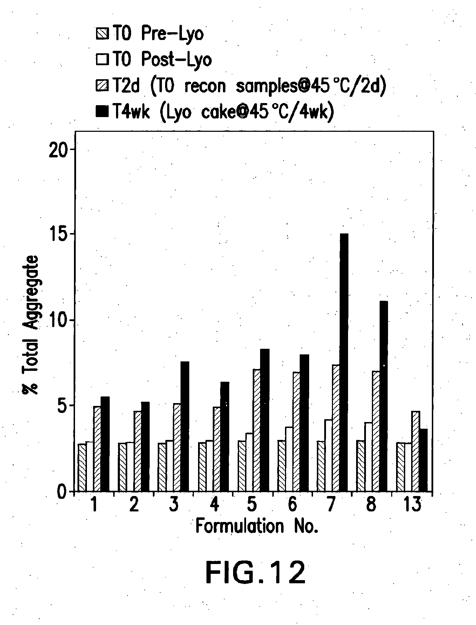

FIGURE 12 shows the percent total aggregate present in various formulations at 100 mg/ml 10G5-6 (Example 2), as measured before and after lyophilization by SEC-HPLC. For comparison purposes, the lyophilized cakes stored at 45° C for 4 weeks (T4wk) and the T0 reconstituted samples at 45° C for 2 days (T2d) are also shown.

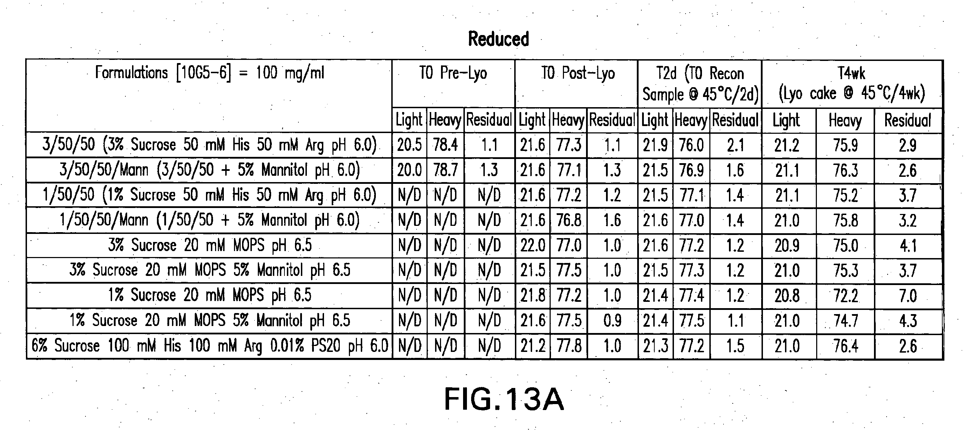

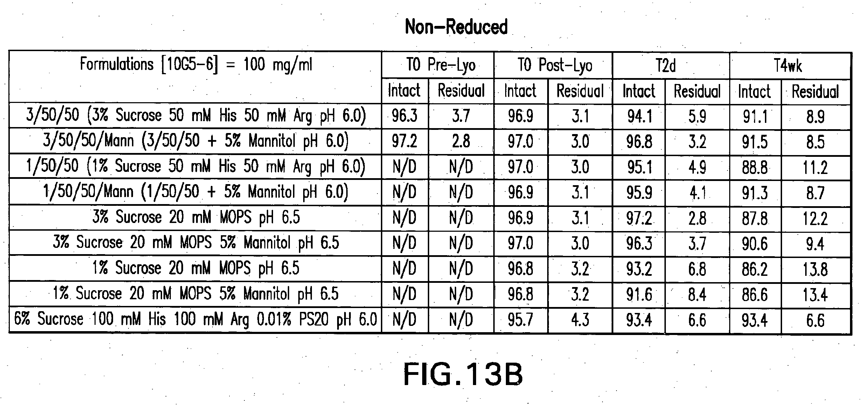

FIGURE 13 shows results of an analysis of the fragmentation and aggregates present in various formulations at 100 mg/ml 10G5-6 (Example 2), as measured before and after lyophilization using caliper SDS-PAGE. The results are shown for the light and heavy chains under reduced conditions (panel A). The residual percent clipping/fragments of the samples is also shown. Under non-reduced conditions, the percent intact monomer and the percent residual clipping/fragments is shown (panel B). SDS caliper data showed a similar trend to SEC data in that it shows a high percent of intact monomer for all formulation before and after lyophilization, suggesting minimal or no adverse effect of lyophilization on the stability of 10G5-6. For comparison purposes, the lyophilized cakes stored at 45° C for 4 weeks (T4wk) and the T0 reconstituted samples at 45° C for 2 days (T2d) are also shown. N/D = not determined.

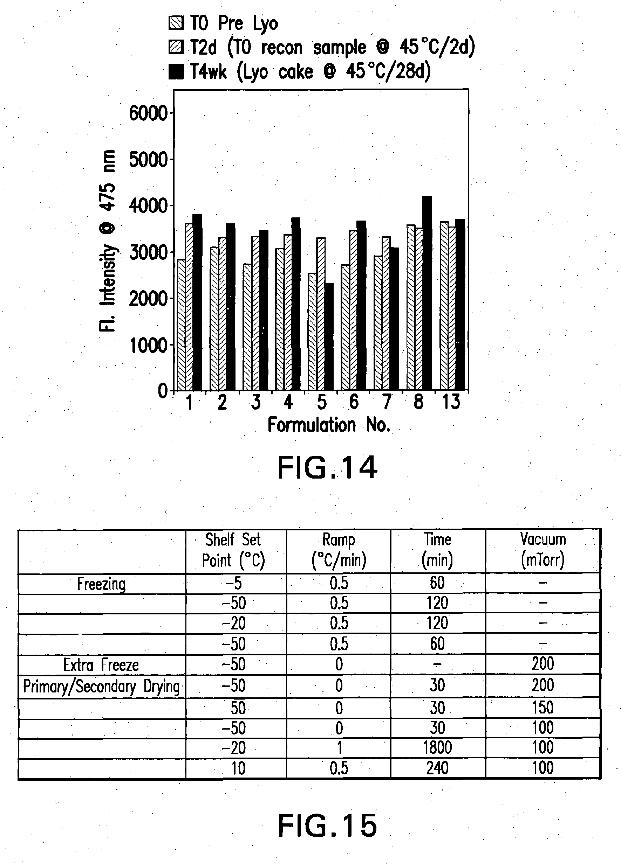

FIGURE 14 shows the results of extrinsic fluorescence measurements of the test formulations (at 100 mg/ml protein concentration) described in Example 2, using 20x excess ANS. The data was normalized to protein concentrations.
FIGURE 15 shows the lyophilization cycle parameters used for the secondary screen, as described in Example 3.

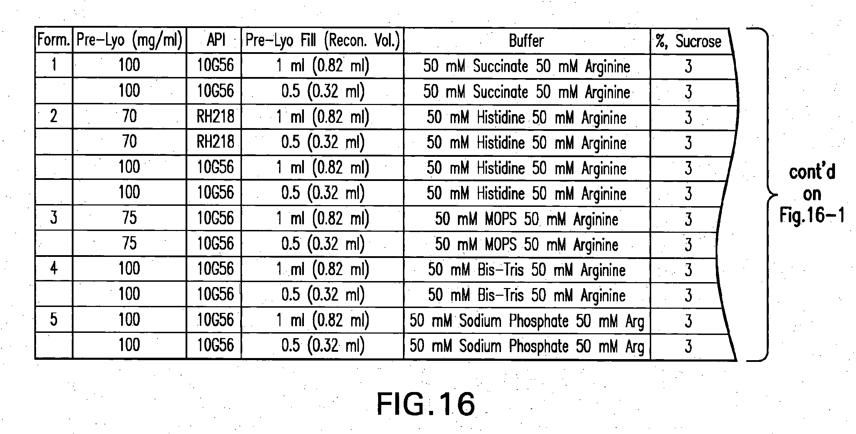

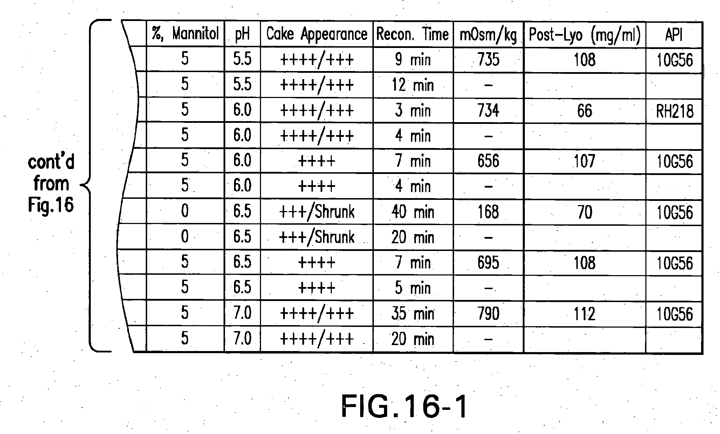

FIGURE 16 shows the reconstitution time and cake appearance, osmolality and concentration post lyophilization for formulations containing 70-100 mg/ml of 10G5-6 and RH2-18 (Example 3). Vials with 1 ml samples were reconstituted with 820 µl of SWFI and 0.5 ml samples were reconstituted with 320 µl SWFI. (Recon. Time). Osmolality measurements were also performed on the advanced Micro Osmometer Model 3350 calibrated using 50 mOsm/kg and 850 mOsm/kg standard and verified using Clinitrol™ 290 Reference Solution (Advanced Instruments, Inc., Norwood, MA). The following symbols were used to denote appearance of the cake: (1) ++++ elegant cake, no cracking; (2) +++ less intact, slight cracking; (3) ++ collapsed cake, more cracking; and (4) + completely collapsed cake.

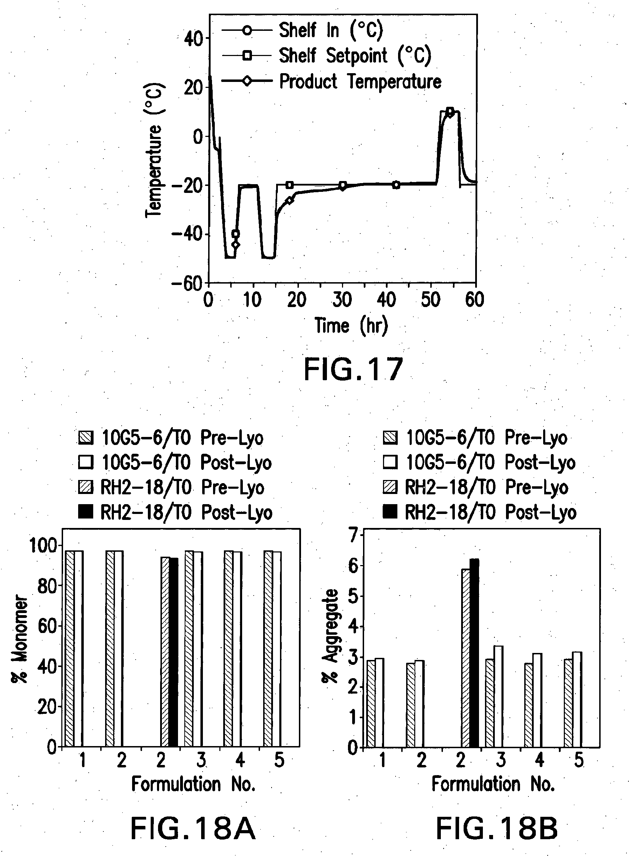

FIGURE 17 shows the product temperature profile during lyophilization of the test formulations described in Example 3. Also shown is the temperature of the shelf and the shelf set point temperature (°C).
FIGURE 18 shows the percent monomers (panel A) and total aggregation (panel B) in test formulations (Example 3) comprising 70-100 mg/ml 10G5-6 or RH2-18, as measured before and after lyophilization by SEC-HPLC.

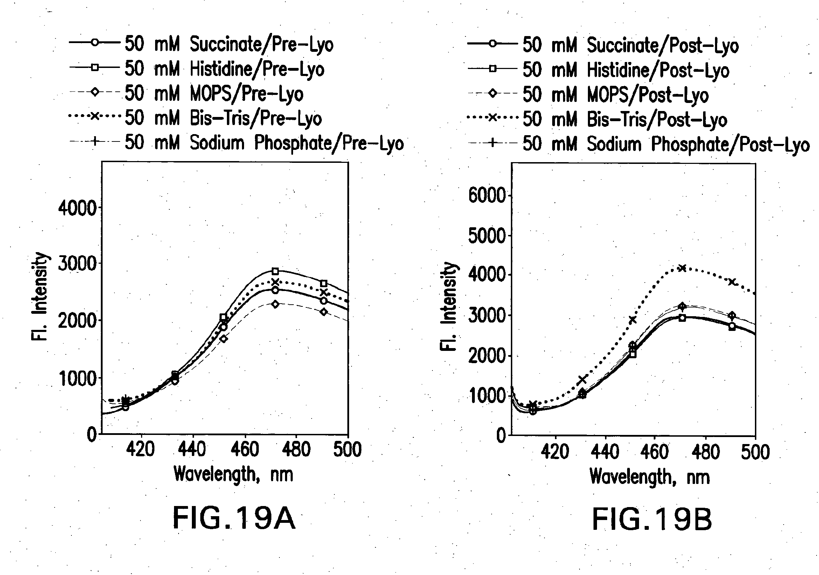

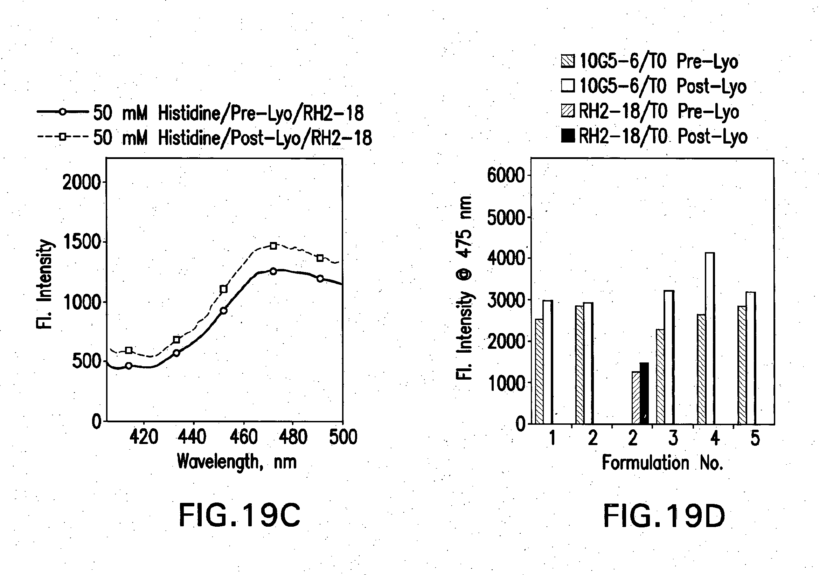

FIGURE 19 shows the results of extrinsic fluorescence measurement of the samples described in Example 3 using 20x excess ANS. The λex was 375 nm. The data was not normalized to protein concentrations, which vary slightly between pre- and post-lyophilization.

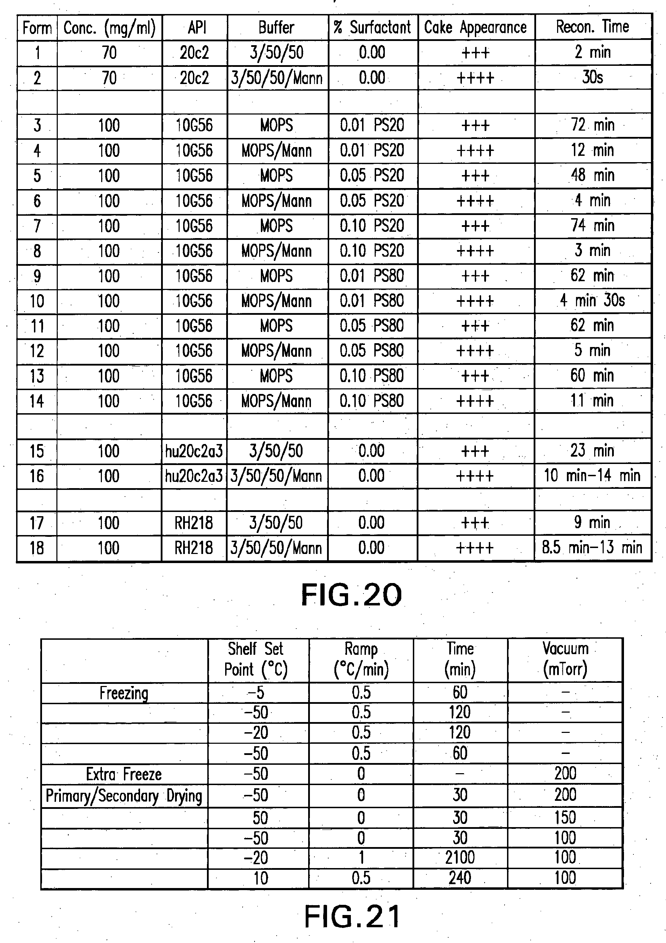

FIGURE 20 shows the reconstitution time and cake appearance for test formulations comprising buffer 3/50/50 (3% Sucrose 50 mM Histidine 50 mM Arginine pH 6.0) and MOPS (3% Sucrose 20 mM MOPS pH 6.5), in presence and absence of (Mann) 5% Mannitol, with 0-0.1% of Tween®20 (PS20) or Tween®80 (PS80) (see Example 4). The concentration of the API in the test formulations was either 70 mg/ml 20c2 (formulation numbers 1 and 2), 100 mg/ml of 10G5-6 (formulation numbers 3-14), 100 mg/ml hu20C2A3 (formulation numbers 15-16) or 100 mg/ml RH2-18 (formulation numbers 17-18). The vials were reconstituted with 1 ml SWFI (Recon Time). The following symbols were used to denote appearance of the cake: (1) ++++ elegant cake, no cracking; (2) +++ less intact, slight cracking; (3) ++ collapsed cake, more cracking; and (4) + completely collapsed cake.
FIGURE 21 shows the lyophilization cycle parameters used for the formulation screen described in EXAMPLE 4.

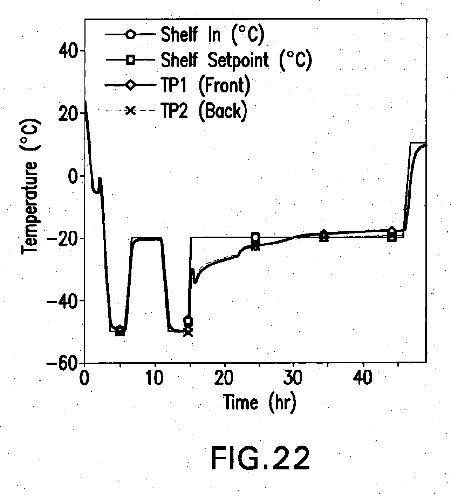

FIGURE 22 shows the product temperature profile of the formulations tested in the formulation screen described in Example 4 during lyophilization in the front (TP1) and back (TP2) of the lyophilization tray.

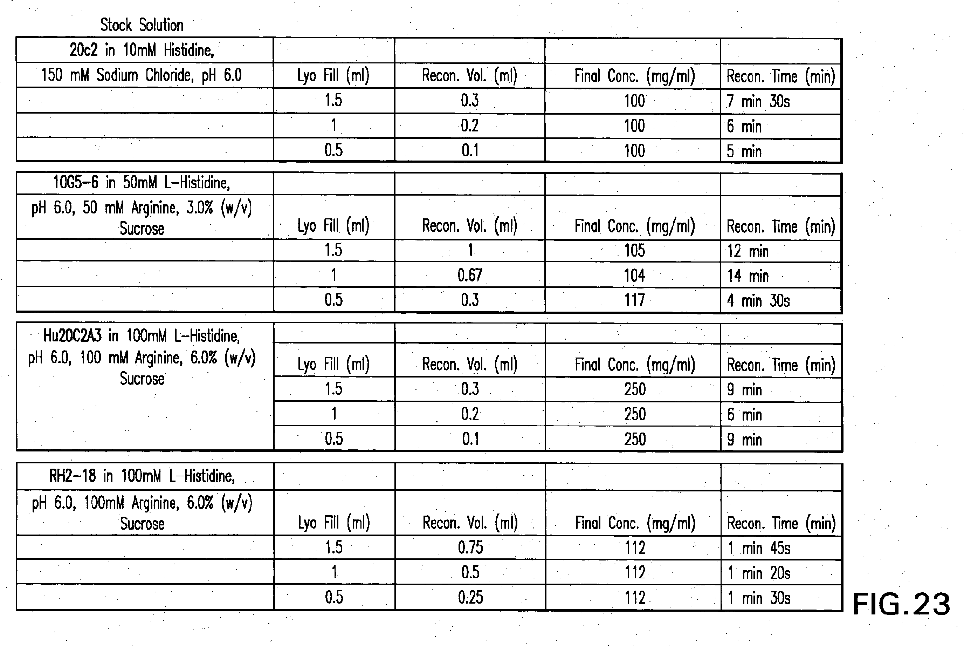

FIGURE 23 shows the affect of pre-lyophilization fill volume on the reconstitution time of test formulations (see Example 5). SWFI was used to reconstitute the formulations.

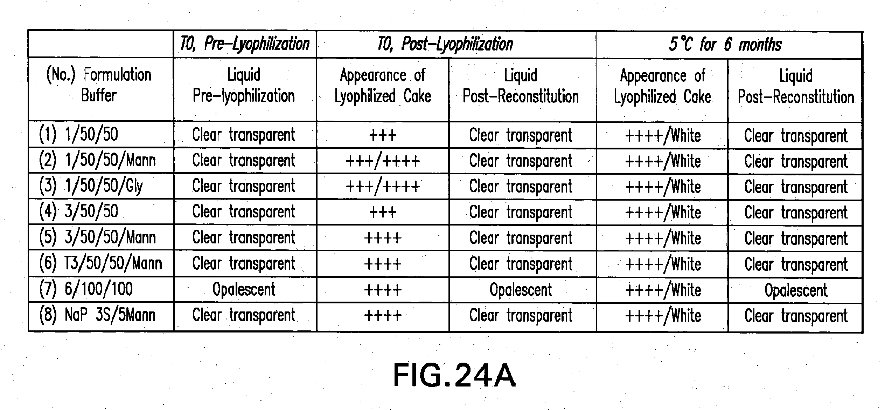

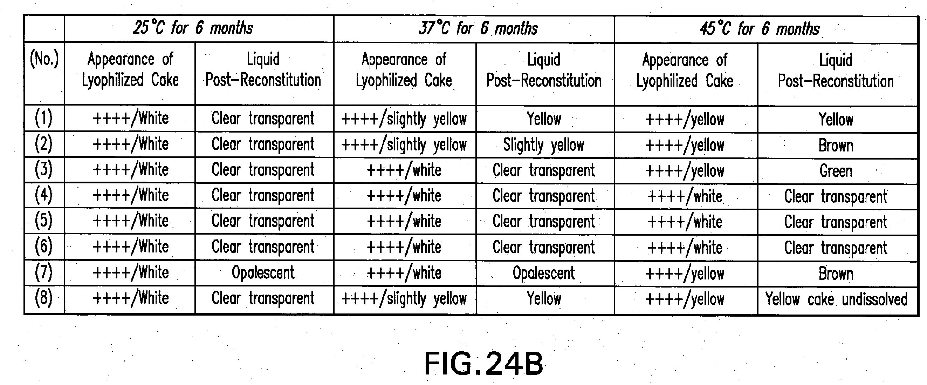

FIGURE 24 shows the cake appearance of the 10G5-6 formulations described in Example 6, prior to lyophilization (pre-lyo, TO), after lyophilization (post-lyo, TO), and after storage for 6 months at various temperatures. Also shown is the appearance of the liquid formulations after reconstitution with SWFI. The following symbols were used to denote appearance of the cake: (1) ++++ elegant cake, no cracking; (2) +++ less intact, slight cracking; (3) ++ collapsed cake, more cracking; and (4) + completely collapsed cake.

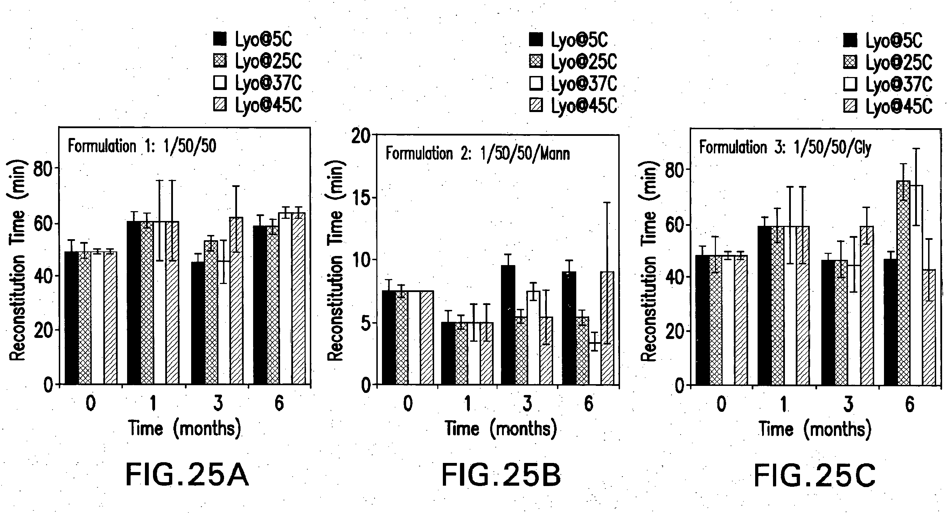

FIGURES 25A-H show the reconstitution times (min.) of the lyophilized formulations described in Example 6 after storage of the lyophilized cakes at 5°C, 25°C, 37°C or 45°C for a period of 6 months. The test formulations were reconstituted with SWFI as described in Example 6. Shown are the reconstitution times for the following formulations: (1) 1/50/50 (panel A); (2) 1/50/50/mann (panel B); (3) 1/50/50/gly (panel C); (4) 3/50/50 (panel D); (5) 3/50/50/mann (panel E); (6) T3/50/50/mann (panel F); (7) 6/100/100 (panel G) and (8) NaP 3S/ 5mann (panel H).

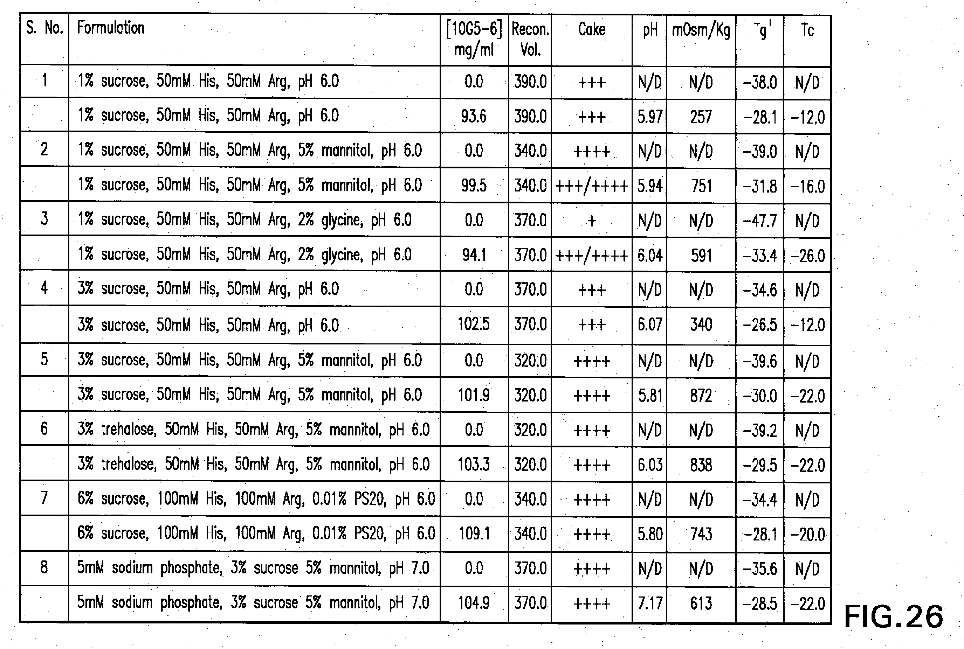

FIGURE 26 shows the cake appearance, pH, glass transition temperatures Tg' (°C), collapse temperature Tc (°C) and osmomolality (mOsm/Kg) of the test formulations described in Example 6 after lyophilization. The concentration of 10G5-6 formulations is shown (∼100 mg/ml) for each formulation. Also shown is data for control formulations corresponding to each of the samples noted, with no antibody. The following symbols were used to denote appearance of the cake: (1) ++++ elegant cake, no cracking, (2) +++ less intact, slight cracking, (3) ++ collapsed cake, more cracking, and (4) + completely collapsed cake. N/D = not determined.

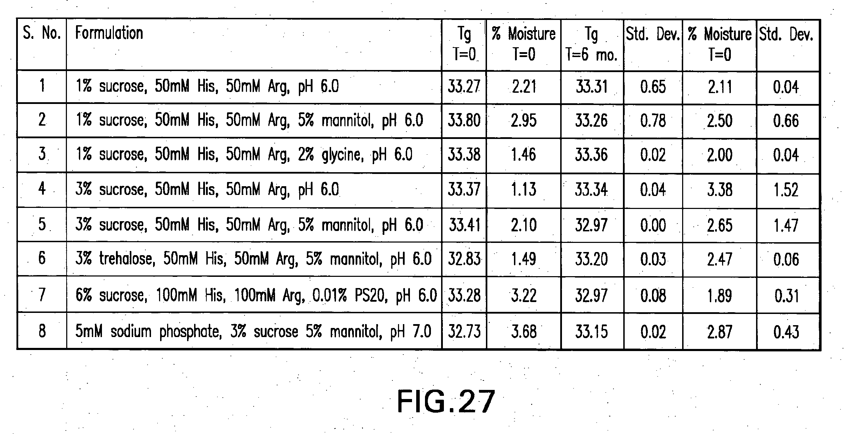

FIGURE 27 provides the glass transition temperatures Tg (°C) and moisture content of each of the test formulations after lyophilization at T0 and after 6 months storage at 5°C. The antibody concentration [10G5-6] was 90 mg/ml for sample no. 1 and 100 mg/ml for all other samples.

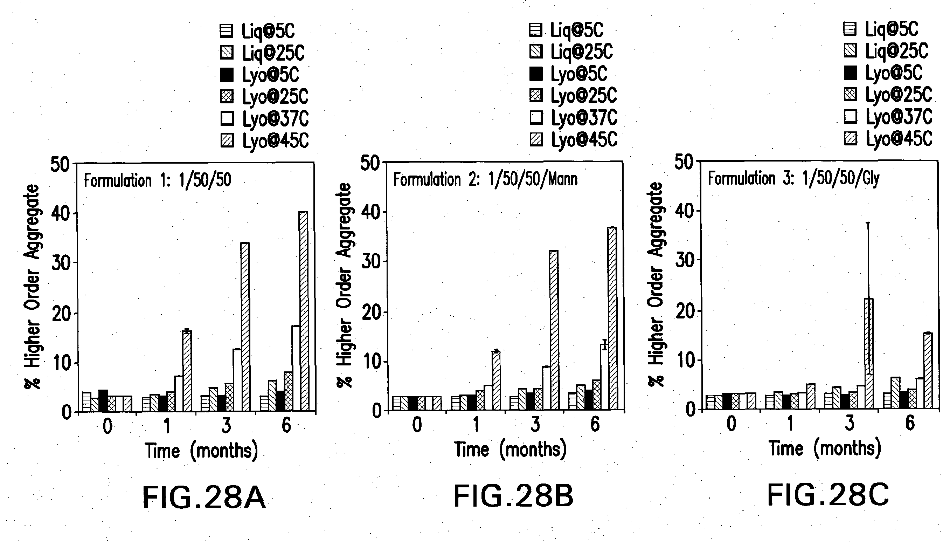

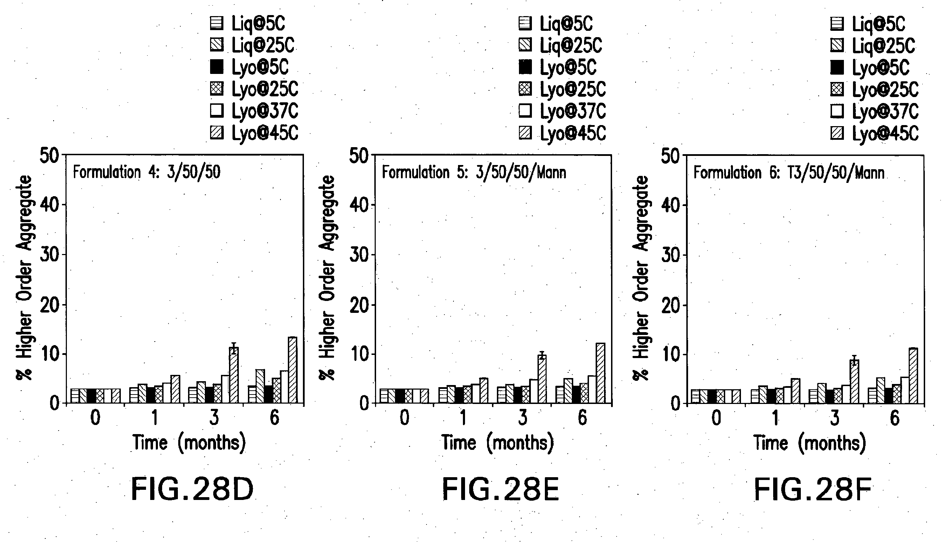

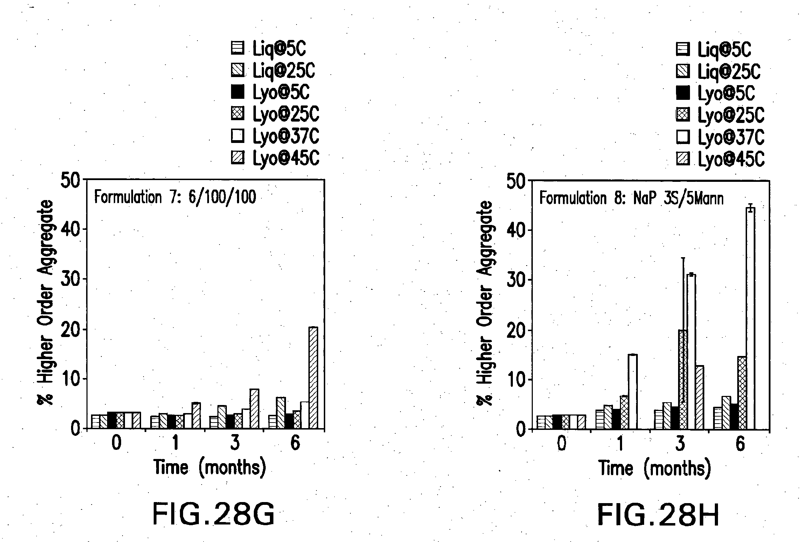

FIGURE 28 shows the percent higher order aggregation in the test formulations described in Example 6 as measured before and after lyophilization by SEC-HPLC following the indicated storage conditions. The test formulations comprised approximately 100 mg/ml 10G5-6 antibody and the following buffers: (1) 1/50/50 (panel A); (2) 1/50/50/mann (panel B); (3) 1/50/50/gly (panel C); (4) 3/50/50 (panel D); (5) 3/50/50/mann (panel E); (6) T3/50/50/mann (panel F); (7) 6/100/100 (panel G) and (8) NaP 3 S/ 5mann (panel H). Data is not provided for the NaP 3S/ 5Mann formulation following storage at 45°C for 6 months due to the failure to dissolve the cake in this formulation.

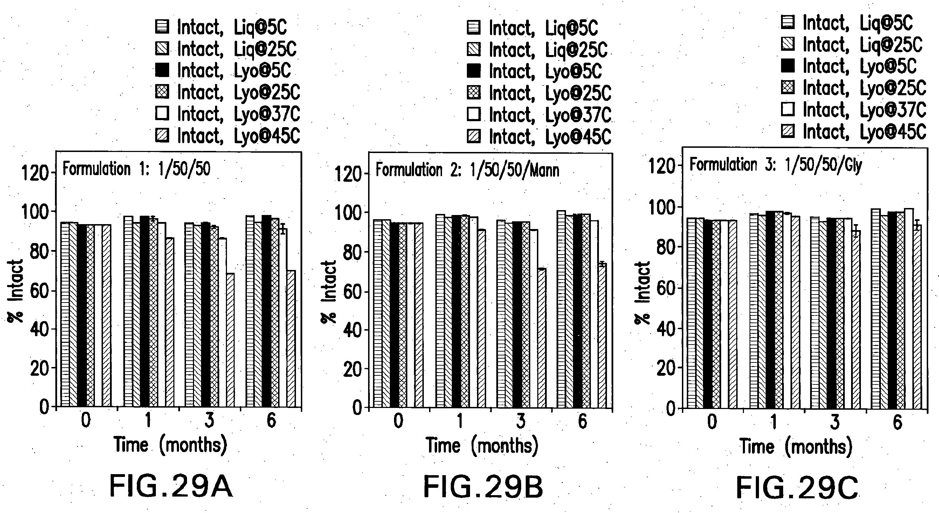

FIGURES 29A-H show the results of caliper SDS-PAGE analysis in which the percent intact monomer present in the test formulations described in Example 6 was measured. The pre and post-lyophilized test samples were analyzed after incubation at 5°C, 25°C, 37°C and 45°C, for 0, 1, 3, and 6 months.

### DETAILED DESCRIPTION OF THE INVENTION

There is a need for methods for producing lyophilized formulations comprising high concentrations of active biological ingredients, which can be reconstituted in a short period of time. Despite the continued interest in high concentration formulations and the associated limitation of longer reconstitution time, as discussed above, prior art methods of attaining high concentration lyophilized formulations focus on the use of low concentrations of protein prior to lyophilization. Methods of obtaining a high concentration lyophilized formulation that utilize a high concentration liquid formulation prior to lyophilization and have a faster reconstitution time would result in an efficient means of delivering an API and improved patient/healthcare provider compliance. Furthermore, such methods would also provide lower production and storage costs due to lower fill/bulk volumes, ultimately resulting in lower cost of goods.

Accordingly, the present invention provides methods for producing stable, high concentration lyophilized pharmaceutical formulations comprising, e.g. 70 mg/ml or higher concentration of protein or antibody, wherein the reconstitution time of the lyophilized formulation is a short period of time, preferably 15 minutes or less. The high concentration lyophilized formulations produced by methods of the invention can employ different API's. Additionally, the lyophilized and reconstituted formulations produced by the methods of the invention are suitable for veterinary or human medical use by oral, pulmonary, and/or parenteral (intravenous, intramuscular, intraperitoneal, or subcutaneous injection) routes of administration. The invention also relates to formulations produced by the methods disclosed herein and their uses thereof.

In accordance with one aspect of the invention described herein, high concentration pharmaceutical compositions are provided wherein said formulations have long term stability and, following lyophilization, can be reconstituted in a short period of time, preferably 15 minutes or less. To this end, one aspect of the present invention is a pharmaceutical composition comprising (i) about 70-250 mg/ml of antibody or about 5 mg/ml to about 60 mg/ml of therapeutic protein or peptide, (ii) about 1% to about 6% w/v sucrose or trehalose, (iii) about 25 mM to about 100 mM histidine, succinate or bis-tris, (iv) about 25 mM to about 100 mM arginine and (v) about 3% to about 8% w/v mannitol, wherein the pH of the composition is about 6.0 to about 7.0. The compositions described herein do not comprise hydrochloric acid.

In additional specific embodiments described herein, the high concentration composition described above comprises about 1%, about 2%, about 3%, about 4%, or about 5% w/v sucrose or trehalose. In alternative embodiments, the compositions described above comprise about 1% to about 5% w/v, about 1% to about 4% w/v, about 1% to about 3% w/v, about 2% to about 6% w/v, about 2% to about 5% w/v, about 2% to about 4% w/v, about 3% to about 6% w/v, or about 3% to about 5% w/v sucrose or trehalose. In some preferred embodiments of this aspect of the invention, the composition described above comprises about 3% w/v sucrose.

In specific embodiments of the invention, any of the pharmaceutical compositions described above comprise about 25 mM to about 100 mM histidine, succinate or bis-tris. In additional embodiments, the concentration of histidine, succinate, or bis-tris in the composition is about 25 mM to about 90 mM, about 25 mM to about 80 mM, about 25 mM to about 75 mM, about 25 mM to about 60 mM, about 25 mM to about 50 mM, about 30 mM to about 90 mM, about 30 mM to about 75 mM, about 30 to about 60 mM, about 30 mM to about 50 mM, about 40 mM to about 90 about, about 40 mM to about 75 mM, about 40 to about 60 mM, or about 40 mM to about 50 mM. In some preferred embodiments, the pharmaceutical composition comprises about 50 mM histidine.

In additional specific embodiments of the invention, any of the pharmaceutical compositions described above may comprise about 25 mM to about 100 mM arginine. In still additional embodiments, the concentration of arginine in the composition is about 25 mM to about 90 mM, about 25 mM to about 80 mM, about 25 mM to about 75 mM, about 25 mM to about 60 mM, about 25 mM to about 50 mM, about 30 mM to about 90 mM, about 30 mM to about 75 mM, about 30 to about 60 mM, about 30 mM to about 50 mM, about 40 mM to about 90 about, about 40 mM to about 75 mM, about 40 to about 60 mM, or about 40 mM to about 50 mM. In specific embodiments, the composition comprises about 50 mM arginine.

In further specific embodiments of this aspect of the invention, any of the high concentration compositions described above may comprise about 3%, about 4%, about 5%, about 6%, about 7%, or about 8% w/v mannitol. In alternative embodiments, the compositions described above comprise about 3% to about 8% w/v, about 3% to about 7% w/v, about 3% to about 6% w/v, about 3% to about 5% w/v, about 4% to about 8% w/v about 4% to about 7% w/v about 4% to about 6% w/v about 4% to about 5% w/v mannitol. In some preferred embodiments of this aspect of the invention, the compositions described about comprise about 5% w/v mannitol.

In alternative embodiments of this aspect of the invention, the high concentration composition comprises sucrose and mannitol, wherein the ratio of sucrose to mannitol is 0.125-3%, alternatively 0.2-3, or 0.2-0.6.

The pH of the pharmaceutical compositions described about is preferably in the range of about 5.5 to about 7.5. In specific embodiments of the invention, the pH of the composition is about 5.5, about 5.75, about 6.0, about 6.25, about 6.5, about 6.75 about 7.0, about 7.25 or about 7.5. In additional embodiments, the pH is about 5.5 to about 7.0, about 5.5 to about 6.5, about 6.0 to about 7.5, about 6.0 to about 7.0, about 6.5 to about 7.0, or about 6.0 to 6.5, or about 6.0 to about 6.75.

Additional embodiments of the present invention provide pharmaceutical compositions as described above which comprise about 70 to about 150 mg/ml antibody. Alternative embodiments are also provided wherein the concentration of antibody is about 70 to about 125 mg/ml or about 70 to about 100 mg/ml. Antibodies that are useful in this aspect of the invention can be of any isotype, including, but not limited to, the IgG1, IgG2, IgG2m4, IgG3 or IgG4. In specific embodiments, the antibody is an IgG1 or IgG2 isotype.

Specific embodiments of this aspect of the invention provide pharmaceutical compositions which comprise (i) about 70 to about 150 mg/ml antibody; (ii) about 2% to about 6% w/v mannitol; (iii) about 1% to about 5% w/v sucrose, (iv) about 25 mM to about 75 mM histidine, and (v) about 25 mM to about 75 mM arginine, wherein the pH of the composition is about 6.0 to about 6.5.

Further provided by this aspect of the invention is a high concentration pharmaceutical composition comprising: (i) about 70 to about 150 mg/ml antibody; (ii) about 3% to about 5% w/v mannitol; (iii) about 1% to about 3% w/v sucrose, (iv) about 25 mM to about 50 mM histidine, and (v) about 25 mM to about 50 mM arginine, wherein the pH of the formulation is about 6.0 to about 6.5.

Also provided by the invention is a high concentration pharmaceutical composition comprising: (i) about 70 to about 150 mg/ml antibody; (ii) about 5% w/v mannitol; (iii) 3% w/v sucrose, (iv) about 50 mM histidine, and (v) about 50 mM arginine, wherein the pH of the formulation is about 6.0.

In an alternative embodiment, the composition comprises: (i) about 70 to about 150 mg/ml antibody; (ii) about 5% w/v mannitol; (iii) about 3% w/v trehalose, (iv) about 50 mM histidine, and (v) about 50 mM arginine, wherein the pH of the formulation is about 6.0.

In an additional embodiment, the composition comprises: (i) about 70 to about 150 mg/ml antibody; (ii) about 5% w/v mannitol; (iii) about 1% w/v sucrose, (iv) about 50 mM histidine, and (v) about 50 mM arginine, wherein the pH of the formulation is about 6.0.

The invention also provides an alternative embodiment in which the high concentration formulation comprises (i) about 70 to about 150 mg/ml antibody; (ii) about 1% to about 6% w/v sucrose, (iii) about 3% to about 8% w/v mannitol, and (iv) about 5 mM to about 100 mM sodium phosphate, wherein the pH of the composition is about 6.0 to about 7.5.

Any of the pharmaceutical compositions described above may optionally comprise about 0.01% to about 0.1 % w/v surfactants. In exemplary embodiments of the invention, the surfactant is a nonionic surfactant selected from the group consisting of: polysorbate 20, polysorbate 80, Brij®35, pluronic® F-68 and Triton®. In some preferred embodiments, the surfactant is polysorbate 20 or polysorbate 80.

The formulations of the present invention may also comprise additional components and pharmaceutically acceptable carriers including, but not limited to an excipient, diluent, stabilizer, buffer, or alternative designed to facilitate administration of the antagonist in the desired format and amount to the treated individual.

In accordance with the invention, it has also been shown that combinations of the formulations described herein with an improved lyophilization cycle and/or reconstitution method results in high concentration, stable lyophilized formulations that can be reconstituted in 30 minutes or less. Preferably, the lyophilized formulations can be reconstituted in 15 minutes or less. In some embodiments of the invention, the dry, lyophilized formulation can be reconstituted in 10 minutes or less. In alternative embodiments, the lyophilized cake can be reconstituted in 5 minutes or less.

The high concentration, lyophilized formulations of the invention are preferably stable for at least 1 month at or below room temperature. The stability of the formulation is tested by various methods used to determine the biophysical properties (such as aggregation using a method to measure turbidity or size exclusion chromatography (SEC)) and chemical properties (such as deamidation using capillary iso-electric focusing or fragmentation using SDS-PAGE) of the API before and after lyophilization and/or after storage conditions. In some embodiments of the invention, the high concentration formulations are stable for up to 6 months at or below room temperature. In still other embodiments, the formulations are stable for up to one year at or below room temperature. In further embodiments the formulations are stable for over a year when stored below room temperature.

Given the complexity of formulation development and the lyophilization process, the development of a method to produce a high concentration lyophilized formulation with a fast reconstitution time requires optimization of individual parameters and their combination to achieve the highest drug quality for the least cost with maximum patience compliance. Therefore, in one aspect, the present invention provides methods which combine high concentration formulations with an optimal freeze-drying process. Some methods of the invention are additionally optimized through the use of optimal fill volumes and diluents, as discussed, *infra.*

### I. Liquid Formulations Prior To Lyophilization.

Liquid formulations suitable for use in the methods of the invention comprise a high concentration of API, a stabilizer or solubilizer, a bulking agent and a buffer which maintains the pH of the formulation in the range of from about pH 4.5 to about pH 7.5. The formulations of the invention may also comprise other excipients and/or surfactants, as described in detail, *infra.*

In order to deliver maximum therapeutic benefits to patients, the most desirable antibody products for subcutaneous (SC) delivery require high protein concentrations (50-250 mg/ml). The main reason for the high concentration stems from the historical bioavailability of 50-60% for SC injections and the expected dose range of 1-2 mg/kg. However, very high protein concentrations may contribute to other properties of the product which would be undesirable, i.e. low injectability due to increased viscosity and higher than physiological osmolality. Therefore, it is preferred to market a product that balances the effects of concentration while maintaining a level of drug that will provide the highest therapeutic results. An ideal product comprises a high protein concentration, low viscosity, and an osmolality similar to physiological conditions. Increased viscosity at high protein concentration may not only make it difficult to extract the product from its container with a syringe, but also to inject the necessary dose into a patient from the syringe (syringeability). This can be frustrating to both the patient and medical professional delivering the treatment. Similarly, it is most favorable to have an antibody formulation with an osmolality similar to physiological condition (∼300 mOsm/kg). Formulations with either high or low osmolalities may result in "stinging upon injection". Thus as a formulator, it is useful to balance the desired concentration, formulation, and osmolality to ensure patient compliance. Advantageously, the formulations produced by the methods described herein have a maximum viscosity and osmolality that are acceptable for subcutaneous delivery, a high concentration of API, and a fast reconstitution time to increase patient compliance.

The formulations for use in the methods of the invention may comprise any therapeutic pharmaceutical/biological or vaccine ingredient suitable for administration to humans or animals in which a high concentration (i.e. 70-250 mg/ml of antibody) of API is desired. A high concentration of API may be desired, for example, when the API is of low potency or when the API is to be delivered subcutaneously. Therefore, a variety of therapeutic proteins or peptides, antibodies and immunologically functional fragments thereof, including single chain antibodies, domain antibodies, and polypeptides with an antigen binding region, useful for therapeutically or prophylactically treating a disease or condition may be utilized in accordance, with the formulations and methods described herein.

In certain embodiments of the invention, the API is an antibody or other immunoglobulin-derived structure with selective binding to a target of interest including, but not limited to, a full length or whole antibody, an antigen binding fragment (a fragment derived from an antibody structure), a derivative of any of the foregoing, a chimeric molecule, a fusion of any of the foregoing with another polypeptide, or any alternative structure/composition which incorporates any of the foregoing. Antibody fragments and, more specifically, antigen binding fragments are molecules possessing an antibody variable region or segment thereof, which confers selective binding to a target antigen. Antibody fragments containing such an antibody variable region include, but are not limited to: a Fab, a F(ab')2_{/} a Fd, a Fv, a scFv, bispecific antibody molecules.

Antibodies that are useful in the formulations and methods of the present invention can be of any isotype, including, but not limited to, the IgG 1, IgG2, IgG2m4, IgG3, or IgG4 subtype. In some embodiments, the antibody is a fully human monoclonal antibody, which preferably does not provoke either antibody-dependent cellular cytotoxicity (ADCC), complement-mediated cytotoxicity (CMC), or form immune complexes to any extent, while retaining its normal pharmacokinetic (PK) properties. In one embodiment, the antibody has an IgG2m4 isotype (*See* U.S. 2007-0148167 filed October 17,2006 and U.S. 2006-0228349 filed October 21,2005).

In some embodiments of the invention, the API of the formulations is a therapeutic protein or peptide, which is preferably present at a high concentration. Such a high concentration of therapeutic protein is typically present in the range of about 5 mg/ml to about 60 mg/ml. One skilled in the art can readily determine an effective concentration of such a therapeutic protein or peptide.

Alternatively, the methods of the invention may be useful for preparing therapeutic and prophylactic vaccines, useful for stimulating an immune response against a desired antigen. In such embodiments, the API may be in the form of a virus-like particle, a live-attenuated virus, a killed virus, a toxoid, a subunit-based vaccine, a conjugate vaccine, or a recombinant DNA vaccine.

The total amount of protein or antibody in the formulations of the invention is a therapeutically effective amount. Such amount will depend, for example, upon the therapeutic context and objectives. One skilled in the art will appreciate that the appropriate dosage levels for treatment, according to certain embodiments, will thus vary depending, in part, upon the API itself, the indication for which the formulation is being used, the route of administration, and the size (body weight, body surface or organ size) and/or condition (the age and general health) of the patient or animal. In some cases, a clinician may titer the dosage and modify the route of administration to obtain the optimal therapeutic effect.

A buffer may also be included in the formulations useful in the methods of the invention. When a buffer is employed, the pH of the buffer is in the range of about 4.5 to about 7.5, prior to lyophilization. In some preferred embodiments of the invention, the pH of the buffer is in the range of about 5.5 to about 7.0. In alternative preferred embodiments, the pH of the buffer is in the range of about 6.0 to about 7.0. In still other embodiments, the pH is in the range of about 6.0 to about 6.5. In some preferred formulations useful for the methods of the invention, the pH is 6.0.

Buffers that may be used for the methods disclosed herein include, but are not limited to: 3/50/50 (3% w/v sucrose, 50 mM histidine, 50 mM arginine), 6/100/100 (6% w/v sucrose, 100 mM histidine, 100 mM arginine), 1/50/50 (1% w/v sucrose, 50mM histidine, 50 mM arginine, MES (2-(N-morpholino)ethanesulfonic acid), histidine, citrate phosphate, MOPS (3-(N-morpholino)propanesulfonic acid), HEPES (4-(2-hydroxyethyl)-1-piperazineethanesulfonic acid), MOPSO, bis-tris, tris, sodium phosphate, potassium phosphate, succinate, TAPS, TES, and PIPES. In some preferred embodiments of the invention, the buffer is 3/50/50,1/50/50, MOPS, MES, sodium phosphate, postassium phosphate, 6/100/100, succinate, or bis-tris.

The buffers used in the methods of the invention should be present in a concentration of about 5 mM to about 100 mM. In some embodiments, the buffer concentration is in the range of about 10 mM to about 75 mM. In alternative embodiments, the concentration of the buffer is in the range of about 25 mM to about 75mM, about 25 mM to about 100 mM, about 50 mM to about 100 mM, or about 50 mM to about 75 mM.

In some embodiments of the methods described herein, the high concentration formulations comprise a stabilizer or solubilizer. A stabilizer or solubilizer may be added to the formulations to increase or retain the effective amount of API upon storage relative to that in their absence. Said stabilizer or solubilizer can be selected from, but is not limited to, an amino acid, a sugar, surfactant, a polyol, a chelating agent, a preservative, dextran, dextran sulfate, dextran T40, diethanolamine, guanidine, calcium chloride, sodium citrate, albumin, gelatin, PEG, lipids, and heparin. In preferred embodiments of the methods of the invention, the stabilizer or solubilizer is selected from the group consisting of: a sugar, a polyol, and an amino acid.

A specific embodiments, the ratio of stabilizer to bulking agent is about 0.125 to about 3, alternatively, about 0.2-3.0 or 0.2-0.6.

Amino acids and similar organic compounds useful to serve the functions stated above, include, but are not limited to: histidine, arginine, ascorbic acid, aspartic acid, glutamic acid, lactic acid (2-hydroxypropanoic acid), malic acid (hydroxybutanedioic acid), lysine, proline, glycine, and phenylalanine. In some embodiments of the methods of the invention, the formulations comprise histidine and/or arginine. When amino acids are used in the formulation, a concentration of 0.2 to 1.5M is preferred. In some embodiments, the amino acid is present at a concentration of 25 mM, 50 mM, 100mM, 150mM, 200mM, 250mM, 500mM, or 1.0M.

Also useful to serve the function of stabilizer or solubilizer are sugars or polyols, which may be, but are not limited to: sucrose, lactose, trehalose, dextrose, mannitol, sorbitol, glycerol, and cyclodextrin (CD) derivatives such as alpha-CD, 2-OH propyl beta-CD, 2-OH propyl gamma-CD. In some preferred embodiments in which a sugar or polyol is used as a stabilizer or solubilizer, sucrose, lactose, or trehalose is used. The sugar or polyol may be present at a concentration of about 0.5% to about 15% (w/v). In some preferred embodiments, about 1% w/v, about 2% w/v, about 3% w/v, about 4% w/v or about 5% w/v of the sugar or polyol is used. In other preferred embodiments about 1% w/v, about 3% w/v or about 6% w/v of sucrose is used.

The high concentration formulations useful in some of the methods described herein comprise a bulking agent. A bulking agent may be added to the formulations to impart cake structure and to improve the reconstitution time. Bulking agents useful in the methods of the invention include, but are not limited to, mannitol, glycine, sucrose, trehalose, lactose, sorbitol, serine and glycerol. In exemplary embodiments of the inventions defined herein, the bulking agent is mannitol. In alternative embodiments, the bulking agent is glycine of serine.

When mannitol is used as the bulking agent, a concentration of about 0.5 to about 15% (w/v) is preferred. In some embodiments of the invention, a concentration of 0.5% to about 10% mannitol (w/v) or about 0.5% to about 8% w/v is used. In alternative embodiments, a concentration of about 3 to about 15% w/v mannitol is used. In still other embodiments about 3% to about 10% w/v or about 3% to about 8% w/v mannitol is used.

When glycine is used as the bulking agent, a concentration of about 0.5% to about 5% (w/v) is preferred. In some embodiments, about 1% w/v, about 2% w/v, about 3% w/v, about 4% w/v, or about 5% w/v glycine is used.

As stated above, certain embodiments of the methods herein comprise a formulation which comprises 3% w/v or more mannitol. When such concentration of mannitol is used, an additional stabilizer or solubilizer (e.g. sucrose) may be omitted if at such high concentration of bulking agent the formulation is sufficiently stable. However, in some cases, it may be advantageous to use an excess of mannitol (i.e. >3%) and an additional stabilizer (e.g. sucrose) to confer additional storage stability, depending on the API and the stability profile of the formulation.

The formulations of the present invention may optionally comprise a surfactant (surface active agent). Surfactants may be added to reduce and/or prevent aggregation or to prevent and/or inhibit protein damage during processing conditions such as purification, filtration, freeze-drying, transportation, storage, and delivery. Surfactants that are useful in the formulations of the invention include, but are not limited to: nonionic surfactants such as polyoxyethylene sorbitan fatty acid esters (polysorbates, sold under the trade name Twee® (Uniquema Americas LLC, Wilmington, DE)) including polysorbate-20 (polyoxyethylene sorbitan monolaurate), polysorbate-40 (polyoxyethylene sorbitan monopalmitate), polysorbate-60 (polyoxyethylene sorbitan monostearate), and polysorbate-80 (polyoxyethylene sorbitan monooleate); polyoxyethylene alkyl ethers such as Brij® 58 (Uniquema Americas LLC, Wilmington, DE) and Brij® 35; poloxamers (e.g., poloxamer 188); Triton® X-100 (Union Carbide Corp., Houston, TX) and Triton® X-114; NP40; Span 20, Span 40, Span 60, Span 65, Span 80 and Span 85; copolymers of ethylene and propylene glycol (e.g., the pluronic® series of nonionic surfactants such as pluronic® F68, pluronic® 10R5, pluronic® F108, pluronic® F127, pluronic® F38, pluronic® L44, pluronic® L62 (BASF Corp., Ludwigshafen, Germany); and sodium dodecyl sulfate (SDS). Cationic surfactants may also be utilized in the formulations of the invention. Examples of cationic surfactants useful in the invention include, but are not limited to: benzalkonium chloride (BAK), benzethonium chloride, cetramide, cetylpyridinium chloride (CPC), and cetyl trimethylammonium chloride (CTAC), primary amines, secondary amines, tertiary amines, and quaternary amine salts.

In exemplary embodiments of the invention, the surfactant is a nonionic surfactant selected from the group consisting of: polysorbate 20, polysorbate 80, Brij®35, pluronic® F-68 and Triton®. In some preferred embodiments, the surfactant is polysorbate 20 or polysorbate 80.

The amount of surfactant to be included in the formulations of the invention is an amount sufficient to perform the desired function, i.e. a minimal amount necessary to prevent protein aggregation, to prevent or inhibit the formation of particulates, to reduce the amount of aggregation of the lyophilized protein or antibody after reconstitution to an acceptable level, to allow ease of reconstitution or to provide a stability advantage during shipping and/or processing. Typically, the surfactant is present in a concentration of from about 0.001% to about 0.5% (wt/vol). In preferred embodiments of this aspect of the invention, the surfactant is present in the formulation (prior to lyophilization) in an amount from about 0.005% to about 0.4%; in more preferred embodiments, the surfactant is present in an amount from about 0.01 % to about 0.3%. In particularly preferred embodiments, the surfactant is present in an amount from about 0.015% to about 0.1%. In alternate preferred embodiments, the surfactant is present in an amount from about 0.05% to about 0.1%.

As dictated by the need of the particular formulation, one or more additional excipients may be added to the formulations which are utilized in the methods of the invention. Such additional excipients do not affect the reconstitution time of the lyophilized formulations produced by the methods herein. One example of an additional excipient that may be added to the formulations of the invention include adjuvants, which may be added to increase the immune response of the patient's immune system to the API. Other excipients that may be added to the formulations include, but are not limited to: a buffer, a stabilizer, a solubilizer, a tonicity modifier, a chelating agent, a preservative, dextran, dextran sulfate, dextran T40, diethanolamine, guanidine, calcium chloride, sodium citrate, albumin, gelatin, polyethylene glycol (PEG), lipids, and heparin. The skilled artisan is readily able to determine which additional excipients should be included in a desired pharmaceutical formulation, dependant on its function in the formulation, as well as the projected mode of administration, dosage, and other factors such as the expected storage time and temperature of the formulation. The skilled artisan recognizes that the amount of the additional excipients may vary, and can readily determine a proper amount that is both safe for administration to humans or animals and effective for the desired function. Typically, an additional excipient may be present at a concentration of about 10 to about 500 mM.

### II. Lyophilization Cycle

It has been shown that a high concentration formulation, such as the high concentration formulations disclosed herein have a faster reconstitution time post-lyophilization when combined with an optimal lyophilization cycle. Accordingly, the present invention provides methods for preparing a high concentration, lyophilized pharmaceutical formulation wherein the lyophilized formulation can be reconstituted in less than 15 minutes, which methods combine a high concentration formulation described herein with an optimal lyophilization cycle.

The process of lyophilizing (also known as "freeze-drying") formulations comprises three stages, namely (1) freezing, (2) primary drying and (3) secondary drying. The current invention provides an optimal method of lyophilizing high concentration pharmaceutical/biological formulations, which method comprises the steps set forth in more detail, *infra.*

The freezing step of the optimized lyophilization cycles disclosed herein, which is the first step in the process of lyophilization, is carried out at temperatures below Tg' for an amorphous product or below Teu (eutectic temperature) for a product in a crystalline state for a length of time sufficient to allow for transformation of the liquid formulation into a solid state. The length of time required to transform the liquid formulation into a solid state depends in part of the total fill volume in the container used to lyophilize the formulation. When larger fill volumes are used, the length of time required to transform the liquid formulation into a solid state will be longer than when relatively smaller fill volumes are used for a comparable formulation.

At the end of the freezing step, the water present in the liquid formulation is converted into ice and typically less than 20% of water (w/w) is present as liquid. Additionally, the rate of cooling determines the size of ice crystals and the cake structure. Slow freezing, for example, usually results in formation of porous cake with larger ice crystals.

In exemplary embodiments of the invention, the freezing step is carried out at a temperature of from about -60°C to about -30°C. Alternatively, the freezing temperature is from about -55 °C to about -35°C, or from about -50°C to about -40°C. In some preferred embodiments of the methods described herein, the freezing temperature is -50°C.

As stated above, the length of time required for the freezing step is dependant on the fill volume prior to lyophilization. In exemplary methods of the invention, the freezing time is at least about 2 hours, inclusive of an annealing step, as described, *infra.* In some embodiments disclosed herein, the freezing step is carried out for about 5 hours to about 10 hours, from about 6 hours to about 9 hours or from about 6.5 hours to about 7.5 hours.

In the methods of the invention, an annealing step may be incorporated into the freezing step to allow efficient crystallization of the crystalline bulking agent (such as glycine or mannitol). An annealing step comprises raising (cycling) the shelf set point temperature of the lyophilizer to a temperature of around -30°C to about -10°C. The temperature should be raised at a rate ("ramp") of about 0.05 to about 2°C/minute. In some methods of the invention, the ramp rate of the annealing step is 0.5 °C/minute.

To achieve a high crystallization rate and complete crystallization, the annealing temperature is held between the Tg' of the amorphous phase and Teu of the bulking agent. The optimal time required for the annealing step is dependent on the type of bulking agent used and the mass ratio of bulking agent to other solutes. It should be noted that annealing above Tg' results in growth of ice crystals thereby decreasing the product resistance to the flow of water vapor eventually resulting in shorter primary drying times. The product specific surface area, however, is reduced as a result of the growth of ice crystals which may result in increased residual moisture content during the secondary drying stage or may require a longer secondary drying period.

In exemplary embodiments of the invention, the annealing step comprises raising the shelf-set point temperature of the freeze-dryer to a temperature in the range of about -30°C to about -10°C. In some embodiments, the temperature is raised about 20 to about 40 degrees higher than the freezing temperature and subsequently held at the higher temperature for a length of time of at least about 60 minutes. In some preferred embodiments, the annealing step comprises raising the temperature about 30 degrees from the freezing temperature. In alternative embodiments of the methods of the invention, the annealing step comprises raising the temperature to about-20°C.

The annealing step is carried out for at least as long as is required to raise the temperature from the initial freezing temperature to the annealing temperature, given the rate of temperature change/ramp. For example, if the initial temperature is -50°C and the annealing temperature is -20°C (shelf set point), a ramp of 0.5°C/minute would require a minimum annealing time of 60 minutes at -20°C shelf set point. Additional time may be added to this step to ensure sufficient crystal growth of the bulking agent and/or ice. In exemplary embodiments of the methods herein, the annealing step is carried out for a period of around 60 to about 150 minutes. In other embodiments, the annealing step is carried out for about 80 minutes to about 140 minutes, from about 100 minutes to about 130 minutes, or from about 110 minutes to about 125 minutes. In some embodiments the annealing step comprises raising the shelf set point temperature from about -50°C to about -20°C, with a ramp rate of 0.5°C/minute, for 120 minutes.

The methods of the present invention may optionally comprise a "pre-freezing" step prior to the freezing step discussed above. The pre-freezing step comprises holding the containers (e.g. vials) in the freeze-dryer, with the shelf set point temperature at about -10°C to about -25°C for at least about 30 minutes or a time sufficient to provide a homogeneous temperature for the containers. In some cases, a longer pre-freezing time may be preferred.

The second step of the freeze-drying process consists of primary drying. The primary drying step can be optimized based on the particular formulation, the shelf temperature, the container closure and the chamber pressure. Generally, the product temperature should be several degrees below Tc and/or Tg' to avoid collapse. Additionally, the methods of the present invention utilize formulations comprising a bulking agent, which also reduces the likelihood of collapse since the collapse temperature is close to the eutectic temperature of the bulking agent (e.g., Teu for mannitol∼ -3°C while Tg' of sucrose is ∼ -34°C. Therefore at a high mannitol to sucrose ratio the Tc would be close to -5°C). Notably, product/formulation temperature during drying is usually 5-40°C lower then shelf temperature and can depend on many variables, including chamber pressure, heat transfer coefficient of the vials, the freeze dryer unit, and the formulation. As an approximate guideline, for every 5°C change of shelf temperature the product temperature changes by 1-2°C. The product temperature, as monitored using a thermal probe, is typically higher at the front and side or back and colder in the interior because of radiative heat transfer from the chamber walls and the freeze-dryer's door.

To improve the rate of ice sublimation, the drying step of the methods disclosed herein is carried out at low pressure (50-200 mTorr). Very low pressure results in larger heterogeneity in heat transfer and might also cause contamination of products with volatile stopper components or pump oil. Thus, an optimal shelf pressure allows for a high sublimation rate with homogenous heat transfer. In some embodiments of the invention herein, the vacuum pressure is set to a pressure (mTorr) from about 100 to about 200.

At the end point for primary drying, the product temperature increases to shelf temperature. Because not all vials finish drying at the same time, an additional soak time of at least about 20% may be added to the primary drying time of the methods of the invention. Such additional time may be added to account for the temperature variance across the shelf.

Secondary drying, the last stage of freeze-drying, removes the water from solute phase by desorption. Secondary drying is achieved at a slow ramp rate to avoid collapse of amorphous product (∼ 0.1 °C/min) while a higher ramp rate might be used for crystalline product (∼ 0.5°C/min). Also, recommended protocol is drying of product at high temperature for a short period of time than at low temperatures for longer times. Desorption rate is very sensitive to product temperature and does not depend on chamber pressure at pressure less than 200 mTorr. Secondary drying also depends on the solute concentration with higher solute concentration (>10%) resulting in longer drying times at a given temperature.

In accordance with the invention herein, it has been determined that one optimal lyophilization cycle includes the steps of (a) freezing the formulation at a first temperature for a length of time sufficient to transform the liquid formulation into a solid state, wherein the first temperature is in the range of about -55°C to about -25°C; (b) annealing by freezing the formulation at a second temperature, wherein the second temperature is in the range of about - 30°C to -5°C; (c) drying the formulation at a third temperature for a length of time sufficient for the formulation temperature to reach within 10% of the shelf temperature; wherein the third temperature is in the range of about -30°C to about -40°C, and wherein the drying step is performed under 5-200 mTorr of pressure; and (d) drying the formulation at a fourth temperature for a length of time sufficient for the formulation temperature to reach within 10% of the shelf temperature; wherein the fourth temperature is in a range of from about 5°C to about 60°C, to produce a dry cake; wherein the dry cake can be reconstituted in about 15 minutes or less to produce a high concentration reconstituted formulation. For sake of clarity, it is understood that the invention embraces methods in which additional steps are added before, after, or between the method steps mentioned above.

The optimal lyophilization cycle described above is useful for freeze-drying the formulations described herein, particularly formulations comprising a high concentration of API (e.g. between 70 and 250 mg/ml antibody), a stabilizer or solubilizer selected from the group consisting of: an amino acid, a sugar and a polyol, and a bulking agent, present in a concentration of about 0.5 to about 15% (weight/weight). Other methods for lyophilizing the noted formulation and additional formulations of the invention, may be used as described below.

In alternative embodiments of the methods described herein, the primary and secondary drying steps are combined into a single step. In this aspect of the invention, the initial liquid formulation is prepared and freeze-dried to produce a dry cake using a method comprising the steps of: freeze-drying the high concentration liquid formulation to form a dry cake using a method comprising the steps of: (i) freezing the formulation at a first temperature for a length of time sufficient to transform the liquid formulation into a solid state, wherein the first temperature is in the range of about -55°C to about -25°C; (ii) annealing by freezing the formulation at a second temperature, wherein the second temperature is in the range of about -30°C to -5°C; (iii) drying the formulation at a third temperature for a length of time sufficient for the formulation temperature to reach within ±10% of the shelf temperature, wherein the third temperature is in the range of about -40°C to about 30°C, and wherein the drying step is performed under 5-200 mTorr of pressure. In this exemplary lyophilization cycle, a secondary drying step is not required because it is combined with the primary drying step. When combining the primary and secondary drying steps, the formulation will take longer to dry when temperatures close to the bottom of the temperature range are selected and, conversely, the formulation will dry faster when higher temperatures are employed. In exemplary embodiments of this aspect of the invention the drying step is carried out at temperatures between -10°C and about 30°C.

In additional alternative embodiments of the methods of the invention, the freeze-drying method comprises the steps of: freeze-drying the high concentration liquid formulation to form a dry cake using a method comprising the steps of: (i) freezing the formulation at a first temperature for a length of time sufficient to transform the liquid formulation into a solid state, wherein the first temperature is in the range of about -55°C to about -25°C; (ii) drying the formulation at a second temperature for a length of time sufficient for the formulation temperature to reach within ±10% of shelf temperature, wherein the second temperature is in the range of about 30°C to about -40°C, and wherein the drying step is performed under 5-200 mTorr of pressure. In this embodiment of the invention, the primary and secondary drying steps are combined into a single step. To decrease drying time, as discussed above, temperatures of about -10°C to about 30°C are used. It is also useful to use a high concentration of bulking agent (e.g. 3-15% w/v) when using this lyophilization method. This method does not use an annealing step as part of the freezing step to crystallize out the bulking agent. Instead, the bulking agent is crystallized during the single freezing step. The lower the temperature used, the longer it will be required to carry out the freezing step in order to both transform the liquid formulation into a solid state and crystallize the bulking agent. For that reason, it may be advantageous in some instances to freeze the formulation at a temperature ranging from about -35°C to about -25°C.

In still other alternative embodiments of the methods of the invention, formulations comprising a high concentration of antibody or therapeutic protein and a high concentration of bulking agent (e.g. about 3% to about 15% (w/v)) are lyophilized using the steps of: freeze-drying the high concentration liquid formulation to form a dry cake using a method comprising the steps of: (i) freezing the formulation at a first temperature for a length of time sufficient to transform the liquid formulation into a solid state, wherein the first temperature is in the range of about -55°C to about -25°C; (ii) drying the formulation at a second temperature for a length of time sufficient for the formulation temperature to reach within ±10% of the shelf temperature, wherein the third temperature is in the range of about 30°C to about -40°C, and wherein the drying step is performed under 5-200 mTorr of pressure; and (iii) drying the formulation at a third temperature for a length of time sufficient for the formulation temperature to reach within ±10% of shelf temperature wherein the fourth temperature is from about 5°C to about 60°C to produce a dry cake.

In some embodiments of this aspect of the invention, a stabilizer or solubilizer is not used in combination with the high concentration of bulking agent in the formulations prior to lyophilization; although in some cases, it may be advantageous to prepare a formulation comprising a high concentration of API, a high concentration of bulking agent, and a stabilizer or solubilizer. As stated above, when not using an annealing step, it may be advantageous to freeze the liquid formulation at a temperature ranging from-35°C to -25°C to decrease the freezing time, relative to freezing at temperatures around -55°C.

After completion of the lyophilization cycles as described herein, a stable high concentrated lyophilized biological formulation results, wherein said formulation can be reconstituted in about 15 minutes or less. Accordingly, the invention also relates to the lyophilized formulations produced by the methods of the invention.

Also provided herein is a kit comprising a container holding a high concentrated lyophilized formulation produced by the methods of the invention, wherein the formulation can be reconstituted in about 15 minutes or less, and instructions for reconstituting the lyophilized mixture with a diluent to produce a high concentration reconstituted liquid formulation. In some embodiments of this aspect of the invention, instructions are included in the kit for reconstituting the lyophilized formulation with a diluent to produce a high concentration antibody formulation with an antibody concentration of 0.5-1.5 times greater than the antibody concentration in the mixture before lyophilization. The kit can further comprise a second container which comprises a diluent.

### III. Container and Fill Volume

Containers useful for the lyophilizing the formulations include, but are not limited to, vials, coupled chamber devices (CCD), syringes, pen devices, and autoinjector devices. Other containment devices such as bags and trays for bulk storage might also be used.

In accordance with the present invention, it has been shown that smaller fill volumes allow for a faster or equivalent reconstitution time post-lyophilization when compared to formulations lyophilized in a higher fill volume (see Example 5). The fill volume is the total volume of liquid in the container used to lyophilize the liquid formulations prepared in accordance with the invention. A 0.5 ml fill, for example, allows for a reconstitution time of 4 min 30 s as compared to 12 min for a 1.5 ml of Ab 10G5-6 formulation at 70 mg/ml in a 3 cc vial. Thus, it is preferred that smaller fill volumes are used for the methods described herein. A small fill volume can be, for example, less than half the fill depth of the container. A fill volume of < 1.5 ml, for example, would be considered a small fill volume for a 3 cc vial.

### IV. Diluent

As stated, *supra,* the lyophilized formulations of the invention can be reconstituted with a diluent in about 15 minutes or less. Diluents useful for reconstituting the lyophilized formulations of the invention include any liquid that is a safe, stable, and pharmaceutically acceptable carrier. In some embodiments of the inventions, the formulations are reconstituted with SWFI and/or BWFI. SWFI containing a stabilizer, a solubilizer, a tonicity modifier, such as NaCl, MgCl₂, or CaCl₂ etc., and mixtures thereof.

In alternative embodiments, the lyophilized formulations of the invention are reconstituted with a second stable pharmaceutical formulation that is stable in liquid form and comprises a different API, relative to the lyophilized formulation being reconstituted, to obtain a combination pharmaceutical product. In such embodiments, the API of the second pharmaceutical formulation can be a small molecule or biological drug product.

In still other alternative embodiments, the lyophilized formulations of the invention are reconstituted with a second pharmaceutical formulation that is stable in liquid form and comprises the same API, relative to the lyophilized formulation being reconstituted. In such embodiments, a higher total amount of drug can be obtained than that amount present in either the lyophilized cake or the diluent.

The volume of diluent used to reconstitute the formulations of the invention is dependent on the intended mode of administration, and the desired final concentration (mg/ml) of the reconstituted formulation. When subcutaneous administration is intended, a diluent volume of less than 1.5 ml is preferable.

Methods of producing reconstituted formulations, by preparing a liquid formulation in accordance with the invention, lyophilizing the liquid formulation using the methods described herein, and reconstituting the lyophilized formulation with a diluent are also encompassed by the invention. In some embodiments of this aspect of the invention, the diluent used is less than or equal to the fill volume used prior to lyophilization. The invention also relates to the reconstituted formulations produced by the methods described herein.

All publications mentioned herein are incorporated by reference for the purpose of describing and disclosing methodologies and materials that might be used in connection with the present invention. Nothing herein is to be construed as an admission that the invention is not entitled to antedate such disclosure by virtue of prior invention.

Having described preferred embodiments of the invention with reference to the accompanying drawings, it is to be understood that the invention is not limited to those precise embodiments, and that various changes and modifications may be effected therein by one skilled in the art without departing from the scope or spirit of the invention as defined in the appended claims.

The following examples illustrate, but do not limit the invention.

### EXAMPLE 1

### Initial Screening of Formulations

The study described herein was undertaken to develop a broadly applicable strategy to obtain lyophilized formulations suitable for veterinary and/or human medical use comprising a high concentration of protein or antibody (e.g. from about 70 to about 250 mg/ml of antibody or from about 5 to about 60 mg/ml of therapeutic protein or peptide), wherein the lyophilized formulations have a reconstitution time of less than 15 minutes. To make progress towards this goal, a formulation screen was performed using a humanized monoclonal antibody that binds to human interleukin 13 receptor alpha 1 (IL-13Rα1, hereinafter "10G5-6") as amodel protein (See Nash et al., WO 2008/060813, published May 22, 2008, which is herein incorporated by reference in its entirety). 10G5-6 is an affinity optimized variant of parental antibody 10G5 (Nash *et al., supra*)*.* The amino acid sequences of the variable heavy and variable light chain regions of 10G5-6 are disclosed herein as SEQ ID NO:1 and SEQ ID NO:2, respectively.

10G5-6 is an antibody of isotype IgG2m4, which is a derivative of IgG2 that is useful to decrease Fc receptor engagement, C1q binding, unwanted cytotoxicity or immunocomplex formation while maintaining both the long half-life and pharmacokinetic properties of a typical human antibody (for further information, *see* Acton et al., US 2006/0228349, which is herein incorporated by reference in its entirety). The basic antibody format of IgG2m4 is that of IgG2, which was shown to possess a superior half-life in experimental models (Zuckier, et al. (1994) Cancer Suppl, 73:794-799). The structure of IgG2 was modified to eliminate C1q binding, through selective incorporation of IgG4 sequences, while maintaining the typical low level of FcγR binding (Canfield and Morrison (1991) J. Exp. Med. 173:1483-1491).

Desired formulations of 10G5-6 were obtained by dialyzing the protein against ten different buffers systems spanning a pH range of 6.0-7.0 (FIGURE 1). The protein formulations were placed into glass tubing vials at three different concentrations (50 mg/ml, 70 mg/ml and 100 mg/ml) at a volume of 1 mL per vial. To prevent product blowout and to improve the product stability, a final concentration of 3% sucrose was maintained in each vial. A liquid portion of each sample was retained at the initiation of the study for turbidity (OD₃₅₀) and size-exclusion chromatography (SEC-HPLC) analysis (see Materials and Methods, Example 7).

The test formulations were lyophilized in a LyoStar II® freeze-dryer (FTS, Stone Ridge, NY) using a conservative cycle (FIGURE 2). During lyophilization, the product temperature profile for samples in the front (TP1), back (TP2) and center (TP3) of the lyophilization tray was recorded (FIGURE 4). The product temperature profile suggested completion of primary drying, as marked by the convergence of shelf temperature and product temperature. A small increase in product temperature above the shelf temperature, which is due to radiative heat transfer, was observed.

Upon completion of the lyophilization cycle, the physical appearance of the dry cakes was recorded (FIGURE 3) prior to reconstituting the cakes in 1.0 mL SWFI or 1.0 mL SWFI containing 0.01 % polysorbate 20 (PS20). The reconstitution time of each formulation was also measured (FIGURE 3). The reconstituted samples were analyzed by OD₃₅₀ (FIGURE 5) and SEC-HPLC (FIGURE 6) to determine whether the lyophilization and reconstitution process had an impact on the stability of the product.

The results show that the reconstitution time increased with increasing protein concentration, under the given experimental conditions. The formulations with the fastest to slowest reconstitution time (left to right) using SWFI as the reconstitution medium at 50 mg/ml, 70 mg/ml, and 100 mg/ml concentrations of 10G5-6 are shown in Table 1. The formulations comprising the 3/50/50 pH 6 buffer had the fastest reconstitution times when 10G5-6 was present at either 70 mg/mL or 100mg/mL. The results also show that in most cases, the reconstitution times were not significantly different when the cakes were reconstituted in 1.0 mL SWFI or 1.0 mL SWFI containing surfactant (PS20, see FIGURE 3).

**Table 1**

| **50 mg/ml** |
|---|
| MOPS pH 6.5>Citrate Phosphate pH 6>Potassium Phosphate pH 7>3/50/50 pH 6>Stock>MOPSO pH 6.5 ∼HEPES>His>Sodium Phosphate pH 7>Citrate Phosphate pH 7> MES pH 6 |

| **70 mg/ml** |
|---|
| 3/50/50 pH 6> Stock >Potassium Phosphate pH 7>MOPS pH 6.6>MOPSO pH 6.5 >Citrate Phosphate pH 6>His pH 6>MES pH 6>HEPES pH 6>Sodium Phosphate pH 7>Citrate Phosphate pH 7 |

| **100 mg/ml** |
|---|
| 3/50/50 pH6>MES pH 6>MOPS pH 6.5>Potassium Phosphate pH 7>citrate Phosphate pH 6>MOPSO pH 6.5>His pH 6>HEPES pH 7>Citrate Phosphate pH 7> Sodium Phosphate pH 7 |

As determined by OD₃₅₀ and SEC-HPLC analyses, the amount of aggregation or formation of particulates was not significantly increased in the lyophilized, reconstituted formulations compared with the pre-lyophilized formulations, indicating that the lyophilization did not affect the stability of the formulations.

### EXAMPLE 2

### Impact of different formulation components, including bulking agent, on reconstitution time.

Four potential buffers from the initial formulation screen (see EXAMPLE 1 and FIGURES 1-6), namely: (1) 3/50/50 pH 6.0 (3% sucrose 50 mM His 50 mM Arg), (2) 20 mM MES pH 6.0 (MES6), (3) 20 mM MOPS pH 6.5 (MOPS65) and (4) 20 mM potassium phosphate pH 7.0 (KP7), were further tested for their ability to reduce reconstitution time in the presence and absence of a bulking agent. The impact of varying the protein concentration and sucrose concentration on reconstitution time was also evaluated. For the secondary screening described in this example, 10G5-6 was again used as a model protein and mannitol was selected as the bulking agent. Desired formulations of 10G5-6 were obtained by dialyzing the protein against eleven different buffers systems spanning a pH range of 6.0-7.0 (FIGURE 7). The protein formulations were placed into glass tubing vials at three different concentrations (50 mg/ml, 70 mg/ml and 100 mg/ml) at a volume of 1 mL per vial. A liquid portion of each sample was retained at the initiation of the study for turbidity (OD₃₅₀), size-exclusion chromatography (SEC-HPLC), capillary iso-electric focusing (cIEF), and caliper SDS-PAGE (sodium lauryl sulfate polyacrylamide gel electrophoresis) analysis (see Example 7).

The test formulations were lyophilized using a conservative cycle (FIGURE 8). During lyophilization, the product temperature profile for samples in the front (TP1), center (TP2, samples without mannitol,) and center (TP3, samples with mannitol) of the lyophilization tray was recorded (FIGURE 10). The product temperature profile suggested the progression of primary drying as marked by the increase in the product temperature towards the shelf temperature with convergence marking the end of primary drying.

After lyophilization, the physical appearance of the dry cakes was recorded (FIGURE 9). The samples were reconstituted with 1 ml SWFI and the reconstitution time of each formulation was measured (FIGURE 9). To determine whether the lyophilization and reconstitution process had an impact on 10G5-6 stability, the reconstituted samples were analyzed by OD₃₅₀ (FIGURE 11), SEC-HPLC (FIGURE 12), cIEF and caliper SDS-PAGE (FIGURE 13) post reconstitution (TO). Additionally, the stability was tested after incubating the lyophilized cake at 45°C for 4 weeks (T4wk) and the T0 reconstituted samples at 45°C for 2 days (T2d).

As determined by OD₃₅₀ and SEC-HPLC analyses, the amount of aggregation or formation of particulates was not significantly increased in the lyophilized, reconstituted formulations compared with the pre-lyophilized formulations in most cases, indicating that the lyophilization did not affect the stability of the formulations. An exception to this observation was formulation 11, comprising 1SKP7 buffer with 50 mg/ml Ab and formulation 9, comprising 3SKP7 buffer with 100 mg/ml Ab, in which aggregation increased post-lyophilization.

Similar to the SEC data, SDS caliper data also showed a high percent of intact monomer for all formulations before and after lyophilization, suggesting minimal or no adverse effect of lyophilization on the stability of 10G5-6. The loss was more significant in T2d and T4wk samples incubated at 45°C, with the greatest loss being observed in 1% sucrose 20 mM MOPS pH 6.0 (96.8% intact at T0 to 93.2% at T2d and 86.2% at T4wk). No significant changes in the amount of intact monomer were observed between formulations in the presence or absence of 5% mannitol, suggesting that mannitol is significantly used in the current formulation as a bulking agent and not a stabilizer. A significant reduction in the loss of intact monomer was observed with increasing sucrose content. For example, a 3/50/50 formulation shows a drop from 96.9% (T0 post-lyo) to 91.1% for a T4wk samples while a 1/50/50 formulation drops from 97.0 % (T0 post-lyo) to 88.8% upon incubation of lyophilized cake for 4 weeks at 45°C. Additionally, a 6/100/100/0.01% PS20 formulation showed little change in percent intact monomer upon incubation for 4 weeks at 45°C (95.7 % at T0 post lyo to 93.4% at T4 weeks). The observed trends suggest the role of sucrose as a stabilizer in the current formulation.

Results from this experiment also showed that the addition of mannitol to the test formulations decreased the reconstitution time when a high concentration (70 mg/ml or 100 mg/ml) of 10G5-6 Ab was present (FIGURE 9). This effect was seen regardless of the buffer tested. Additional experiments were conducted in which reconstitution time was measured using lyophilized formulations comprising 100 mg/ml 10G5-6 in 3/50/50 pH 6.0 buffer with 5% mannitol obtained from either Sigma (Sigma Aldrich Co., St. Louis MO) or MP Biomedicals, LLC (Santa Ana, CA). Results of these experiments showed that the source of mannitol did not have any impact on the reconstitution time of these lyophilized formulations (data not shown). Additionally, the source of mannitol did not have any effect on the stability of the formulations, either before or after lyophilization (data not shown).

Extrinsic fluorescence measurements of the test formulations were also obtained using 20x excess ANS (FIGURE 14). The data was normalized to protein concentrations. Upon binding to hydrophobic site, a large increase in fluorescence quantum yield and the pronounced blue shift of the emission maximum was observed. Smaller changes were observed between samples before and after lyophilization which could be attributed to multiple factors including, but not limited to, different ANS binding sites and their corresponding affinity, structural perturbation in protein etc. These observed differences correlate well with the turbidity measurement.

The glass transition temperatures Tg' and Tg (°C), collapse temperature Tc (°C), viscosity of samples after lyophilization (cP) and osmolality (mOsm/Kg) of the test formulations of 10G5-6 at a concentration of 100 mg/ml are shown in Table 2. These parameters are important for designing an optimal lyophilized formulation. The Tg' for all formulations fell between -24 to -32°C, while the collapse temperature was between -8 to -24°C, thereby allowing the determination of the primary drying product temperature below both Tg' and Tc. Similarly, to ensure maximum stability, it is recommended to store the samples below the Tg (∼ 32 - 38°C, in the present case). As shown in Table 2, the formulations studied had a low viscosity even at 100 mg/ml, suggesting their practical feasibility for developing a product for subcutaneous delivery.

**Table 2**

| Formulation | Tg' | Tg | Tc | Viscosity (cP) | mOsm /Kg |
|---|---|---|---|---|---|
| 3/50/50 (3%Sucrose 50 mM His 50 mM Arg pH 6.0) | -24.6 | 37.2 | -24 | 4.02 | 242 |
| 3/50/50/Mann (3/50/50 + 5% Mannitol pH 6.0) | -29.8 | 36.2 | -24 | 4.51 | 497 |
| 1/50/50 (1%Sucrose 50 mM His 50 mM Arg pH 6.0) | -24.8 | 36.6 | -12 | 3.08 | 181 |
| 1/50/50/Mann (1/50/50 + 5% Mannitol pH 6.0) | -31.1 | 34.6 | -8 | 3.25 | 430 |
| 3%Sucrose 20 mM MOPS pH 6.5 | -24.1 | 34.5 | -22 | 2.10 | 150 |
| 3%Sucrose 20 mM MOPS 5% Mannitol pH 6.5 | -29.3 | 36.6 | -22 | 4.22 | 371 |
| 1%Sucrose 20 mM MOPS pH 6.5 | -29.8 | 33.3 | -12 | 3.80 | 90 |
| %Sucrose 20 mM MOPS 5% Mannitol pH 6.5 | -29.1 | 32.5 | -16 | 4.30 | 345 |
| 6%Sucrose 100 mM His 100 mM Arg 0.01% PS20 pH 6.0 | -25.6 | 32.8 | -20 | 2.82 | 463 |

### EXAMPLE 3

### Impact of buffer components on reconstitution times and stability of formulations.

The initial and secondary formulation screens suggested 3/50/50/Mann pH 6.0 (3% sucrose 5 % mannitol 50 mM His 50 mM Arg) may be useful as a platform that could be used to attain high concentration formulations of a desired API with a fast reconstitution time (Examples 1 and 2). To determine the role of histidine in reconstitution and stability of proteins upon lyophilization, histidine in the 3/50/50/Mann buffer was substituted with 50 mM succinate, 50 mM bis-tris, and 50 mM sodium phosphate, respectively (Table 3) in the test formulations. Also studied was 3% sucrose 50 mM MOPS pH 6.5 as a positive control (no mannitol, buffer 3, Table 3).

**Table 3. Buffers for lyophilization screen.**

| Buffer | |
|---|---|
| 1 | 50 mM Succinate 3% Sucrose, 5% Mannitol 50 mM Arginine pH 5.5 |
| 2 | 50 mM Histidine 3% Sucrose, 5% Mannitol 50 mM Arginine pH 6.0 |
| 3 | 50 mM MOPS 3% Sucrose, 50 mM Arginine pH 6.5 |
| 4 | 50 mM Bis-Tris 3% Sucrose, 5% Mannitol 50 mM Arginine pH 6.5 |
| 5 | 50 mM Sodium Phosphate 3% Sucrose, 5% Mannitol 50 mM Arginine pH 7.0 |

For formulations tested in accordance with this Example, 10G5-6, as well as RH2-18 were used as model proteins. RH2-18 is a humanized monoclonal antibody specific for dickkopf-1 (Dkk-1), an inhibitor of the osteoanabolic Wnt/LRP5 signaling pathway (*see* An et al., WO 2008/097510, published August 14, 2008, which is herein incorporated by reference in its entirety. The amino acid sequences of the RH2-18 heavy chain and light chain are disclosed herein as SEQ ID NO:3 and SEQ ID NO:4, respectively.

Desired formulations of the antibodies were obtained by utilizing concentrated stock solutions of 10G5-6 and RH2-18 at 70-100 mg/ml and dialyzing the protein against five different buffers systems spanning a pH range of 5.5-7.0 (Table 3). Samples were lyophilized at a starting volume of 1 mL and starting concentration of approximately 70 mg/ml and/or 100 mg/ml. The protein formulations were placed into glass tubing vials at 70 mg/ml and 100 mg/ml at a volume of 0.5-1.0 mL per vial. A liquid portion of each sample was retained at the initiation of the study for size-exclusion chromatography (SEC-HPLC), and extrinsic fluorescence measurement using ANS dye.

The samples were lyophilized using a conservative cycle (FIGURE 15). During lyophilization, the product temperature profile for the samples was recorded (FIGURE 17). As shown in FIGURE 17, the product temperature converged with the shelf temperature and the shelf setpoint, indicating the completion of primary drying.

After lyophilization, the physical appearance of the dry cakes was recorded (FIGURE 16). The samples were then reconstituted with 0.82 or 0.32 mL SWFI for a 1 mL and a 0.5 ml fill, respectively, and the reconstitution time of each formulation was measured (FIGURE 16). To determine whether the lyophilization and reconstitution process had an impact on 10G5-6 stability, the samples were analyzed by SEC-HPLC (FIGURE 18), and ANS fluorescence measurement (FIGURE 19) post reconstitution (TO).

No significant differences in the % monomer level were observed upon lyophilization, as quantitated using SEC-HPLC, suggesting little or no impact of lyophilization on the aggregation of 10G5-6, under the given pH and buffer conditions. To verify that this observation was not limited to the 10G5-6 Ab, similar studies were performed with RH2-18 in the 3% sucrose 50 mM His 50 mM Arg 5 % Mannitol formulation. Comparable results (93.96% pre-Lyo and 93.58 % post-lyo) were obtained from SEC-HPLC, suggesting the current approach supports the use of different APIs.

Also, results from the above experiments show that in certain cases, a lower fill volume provided a faster reconstitution time. For example, as the fill volume was lowered from 1 ml to a 0.5 ml, the reconstitution time of 10G5-6 in 50 mM MOPS 50 mM Arginine 3% sucrose pH 6.5 significantly dropped from 40 min to 20 min, respectively.

The results of extrinsic fluorescence measurement of the samples described in Example 3 using 20x excess ANS provides an insight into the tertiary structural changes of the protein upon lyophilization. The data was not normalized to protein concentrations, which vary slightly between pre- and post-lyophilization. Upon binding to hydrophobic site, a large increase in fluorescence quantum yield and the pronounced blue shift of the emission maximum was observed. Smaller changes were observed between samples before and after lyophilization, which could be attributed to multiple factors including, but not limited to, different protein concentration, ANS binding sites and their corresponding affinity, structural perturbation in protein etc. The observed differences between pre- and post-lyophilization samples in formulations 1, 2 and 5 were well within experimental error, suggesting little or no structural changes occurred in the protein as a result of lyophilization.

Glass transition temperatures Tg' and Tg (°C) for formulations containing 70-100 mg/ml of 10G5-6 and RH2-18 are shown in Table 4. The Tg fell in the temperature range of -22 to -33 °C while the Tg was in the range of 29 to 50°C. Thus, during lyophilization the primary drying product temperature should be below both the Tg' and Tc. Similarly, to ensure maximum stability it is recommended to store the samples below the Tg.

**Table 4**

| No. | [Conc] mg/ml, Ab | Buffer | % Sucrose | % Mannitol | pH | Tg' | Tg |
|---|---|---|---|---|---|---|---|
| 1 | 100, 10G5-6 | 50 mM succinate 50 mM arginine | 3 | 5 | 5.5 | -32.2 | 36.0 |
| 2 | 70, RH2-18 | 50mM histidine 50mM arginine | 3 | 5 | 6.0 | -32.8 | 33.2 |
| | 100, 10G5-6 | 50mM histidine 50mM arginine | 3 | 5 | 6.0 | -29.4 | 29.1 |
| 3 | 75, 10G5-6 | 50 mM MOPS 50 mM arginine | 3 | 0 | 6.5 | -22.8 | 30.2 |
| 4 | 100, 10G5-6 | 50 mM Bis-Tris 50 mM arginine | 3 | 5 | 6.5 | -32.5 | 49.5 |

### EXAMPLE 4

### Effect of surfactants and different proteins on the reconstitution time of lyophilized formulations

To determine the role of surfactant in reconstitution, varying amounts (0-0.1%) of Tween® 20 (Uniquema Americas LLC, Wilmington, DE) or Tween® 80 were added to test formulations containing 100 mg/ml 10G5-6 antibody in MOPS or MOPS/Mann buffer, lyophilized, and reconstituted (see FIGURE 20). Also, formulations comprising various API's (antibodies) were tested to determine if the beneficial effect of bulking agent seen in previous examples was specific to the 10G5-6 antibody.

The model protein utilized for the initial portion of this study, in which the effect of adding surfactant to the formulations was tested, was the 10G5-6 antibody (see Example 1). For the second portion of this study, in which the role of the API was evaluated, the model proteins were the RH2-18 antibody (see Example 3) and two additional antibodies, 20c2 and hu20C2A3, which differentially recognize multi-dimensional conformations of Aβ-derived diffusible ligands (also known as ADDLs). The 20c2 antibody tested herein is a humanized IgG1 version of the murine anti-ADDL antibody 20c2 (*see* Acton et al., US 2006/0228349, filed on October 21, 2005). The base amino acid sequences of the heavy and light chain variable regions of 20c2 are disclosed herein as SEQ ID NO:7 and SEQ ID NO:8, respectively. In addition, the version of 20C2 tested herein comprised modifications to the CDR3 sequence. Hu20C2A3 is a humanized, affinity matured IgGI version of 20c2 (*see* Kinney et al., WO 2007/050359, international filing date October 17, 2006). The amino acid sequences of the heavy and light chain variable regions of hu20C2A3 are disclosed herein as SEQ ID NO:5 and SEQ ID NO:6, respectively.

For the API study, in addition to one of the antibodies described above, each of the test formulations comprised 3/50/50 (3% sucrose 50 mM His 50 mM Arg pH 6.0), and were tested for reconstitution time and physical appearance in the presence and absence of mannitol (see FIGURE 20). The protein formulations were placed into glass tubing vials at 70-100 mg/ml concentration range and a volume of 1 mL per vial and lyophilized using a conservative cycle (FIGURE 21). The product temperature profile during the lyophilization cycle is shown in FIGURE 22.

The samples were lyophilized using a conservative cycle (FIGURE 21). During lyophilization, the product temperature profile for the samples was recorded. As shown in FIGURE 22, the product temperature converged with the shelf temperature and the shelf setpoint, indicating the completion of primary drying.

Earlier studies (see Example 2) showed that the addition of 5% mannitol to a 20 mM MOPS 3% sucrose pH 6.0 solution significantly decreased the reconstitution time. This example shows that addition of polysorbate 20 or polysorbate 80 (PS20 or PS80, respectively) did not significantly effect the reconstitution time under the given experimental conditions (FIGURE 20). Additionally, the results shown herein indicate that this approach can be applied to other antibodies as exemplified using 20c2, hu20c2a3 and RH2-18.

### EXAMPLE 5

### Effect of fill volume on reconstitution time

To determine if the fill volume had an effect on the reconstitution times of test formulations, compositions comprising Ab 20c2 (20 mg/ml), 10G5-6 (70 mg/ml), HuC2A3 (50 mg/ml), and RH2-18 (56 mg/ml) were formulated, lyophilized, and reconstituted. The specific formulations studies are shown in FIGURE 23. All samples were lyophilized using a conservative cycle (as described in Example 2, and shown in FIGURE 22). The dry cakes were reconstituted with the amount of SWFI required to bring the sample to the desired final concentration (FIGURE 23).

Results show that, for samples comprising a high concentration of Ab (70 mg/ml of 10G5-6 in 3/50/50 buffer), the reconstitution time was affected by the pre-lyophilization fill volume. Specifically, lower fill volumes lead to a quicker reconstitution time. This trend was not seen in the other formulations tested, suggesting that the affect of fill-volume on reconstitution time was limited to high-concentration formulations.

### EXAMPLE 6

### Effect of Excipients on Reconstitution Time and Storage Stability of Lyophilized Formulations.

### (a) Test Formulations

To determine the role of excipients (i.e. buffer, sucrose and mannitol) in reconstitution and stability of protein upon lyophilization, formulation screens were performed with a number of different formulations, each comprising 90-100 mg/mL of 10G5-6 antibody (see Table 5). The first formulation tested comprised 1% sucrose ("1/50/50," see below). Also tested were formulations which comprised 1% sucrose and either mannitol ("1/50/50/Mann") or glycine ("1/50/50/Gly"). To determine if the amount of sucrose had an effect on reconstitution or stability, formulations comprising 3% sucrose, either with mannitol ("3/50/50/Mann") or without ("3/50/50") and 6% sucrose without mannitol ("6/100/100") were tested. Next, 3% trehalose was substituted in place of sucrose in the formulation comprising mannitol ("T3/50/50/Mann"). Finally, the 50 mM His 50 mM Arg, pH 6.0 buffer was substituted with sodium phosphate, pH 7.0 ("NaP 3S/5Mann"). The NaP formulation also had 3% sucrose and mannitol. Desired formulations of the 10G5-6 antibody (described in example 1) were obtained by dialyzing concentrated stock solutions of protein at approximately 90-100 mg/ml against the eight different buffers systems described generally above and shown in Table 5. The buffers spanned a pH range of 6.0-7.0.

**Table 5**

| | Arm | Formulation Buffer |
|---|---|---|
| 1 | 1/50/50 | 90 mg/ml 10G5-6, 1% Sucrose, 50 mM His, 50 mM Arg, pH 6.0 |
| 2 | 1/50/50/Mann | 100 mg/ml 10G5-6, 1% Sucrose, 50 mM His, 50 mM Arg, 5% Mannitol pH 6.0 |
| 3 | 1/50/50/Gly | 100 mg/ml 10G5-6, 1% Sucrose, 50 mM His, 50 mM Arg, 2% Glycine pH 6.0 |
| 4 | 3/50/50 | 100 mg/ml 10G5-6, 3% Sucrose, 50 mM His, 50 mM Arg, pH 6.0 |
| 5 | 3/50/50/Mann | 100 mg/ml 10G5-6, 3% Sucrose, 50 mM His, 50 mM Arg, 5% Mannitol pH 6.0 |
| 6 | T3/50/50/Mann | 100 mg/ml 10G5-6, 3% Trehalose 50 mM His, 50 mM Arg, 5% Mannitol pH 6.0 |
| 7 | 6/100/100 | 100 mg/ml 10G5-6, 6% Sucrose, 100 mM His, 100 mM Arg, 0.01% PS20, pH 6.0 |
| 8 | NaP 3S/5Mann | 100 mg/ml 10G5-6, 5 mM Sodium Phosphate, 3% Sucrose, 5% Mannitol pH 7.0 |

### (b) Lyophilization

The antibody formulations were placed into glass tubing vials at a volume of 0.5 mL per vial. A liquid portion of each sample was retained at the initiation of the study for analysis and the appearance of the liquid was recorded (FIGURE 24A, "T0, Pre-lyophilization").

The test formulations (Table 5) were lyophilized using a conservative cycle as shown in FIGURE 2. During lyophilization, the product temperature profile for the samples was recorded, and it was confirmed that the product temperature converged with the shelf temperature and the shelf setpoint, indicating the completion of primary drying. Furthermore, additional soak time was allowed to ensure completion of primary drying for all the vials.

After lyophilization, the physical appearance of the dried cakes was recorded (FIGURE 24A, "T0, Post-lyophilization"). Under the given experimental conditions, all antibody formulations resulted in the formation of elegant cakes or cakes with slight cracking (FIGURES 24A, 26). The 6/100/100 formulation appeared more turbid than the other formulations pre-lyophilization; however, further characterization revealed that the opalescence was due to the presence of non-proteinacious particles. Furthermore, no significant change in the turbidity of the 6/100/100 formulation was observed at T0 timepoint post-lyophilization.

### (c) Reconstitution

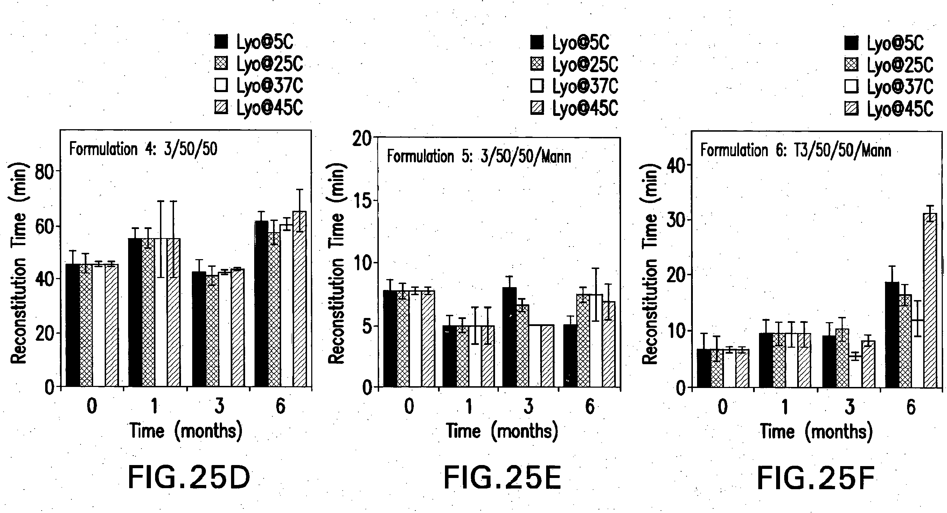

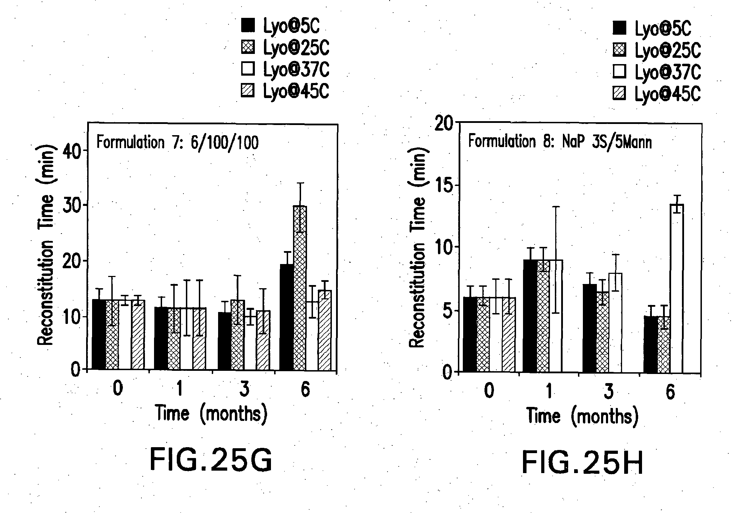

The samples described above were reconstituted with SWFI and the reconstitution time of each formulation was measured (FIGURE 25). The appearance of the liquid following reconstitution was also recorded (FIGURE 24A, "T0, Post-lyophilization"). As noted above, the test samples were lyophilized at a starting volume of 0.5 ml and a starting concentration of the 10G5-6 antibody of approximately 100 mg/ml. The vials were reconstituted to a slightly higher concentration by reconstituting the formulations with slightly lower volumes of SWFI compared to the pre-lyophilized volume (FIGURE 26) to account for volume displacement by the total solid in the cake and also to achieve slightly higher (1-1,6 times) concentration compared to the protein concentration prior to lyophilization. The final concentration of the reconstituted formulations was tested by measuring the absorbance at 280 nm and ranged from 86 to 146 mg/ml. The samples were also analyzed by DSC to study glass transition temperature (Tg') and freeze-drying microscopy to study collapse temperature (Tc) (FIGURE 26)

### (d) Storage Stability

To determine the storage stability of the different formulations (Table 5), samples of the lyophilized cakes for each of the eight formulations were stored for 6 months at various temperatures (5°C, 25°C, 37°C and 45°C), as described below. The cakes were reconstituted after the given storage conditions with the same amount of SWFI as the corresponding formulations at T0 (above) in order to obtain similar final concentrations post reconstitution for all formulations. Several tests were conducted on the lyophilized cakes and reconstituted formulations after storage at each of the temperatures noted above for 1 month, 3 months, and 6 months; including measurement of reconstitution time, MFI, total particle count and SEC-HPLC (Example 6, below).

At the conclusion of each storage period, the appearance of the lyophilized cakes was recorded (FIGURES 24A and B). The cakes were reconstituted as described above following each storage condition (time/temp.) and the appearance of the reconstituted liquid formulations was also recorded (FIGURES 24A and B). The results showed that under stress conditions (higher temp. and longer duration), the color of the lyophilized cakes and also the reconstituted products changed from white to yellowish brown especially after storage at 45°C for 6 months for the 1/50/50, 1/50/50/mann, 1/50/50/gly, 6/100/100 and NaP 3S/5Manm formulations.

The reconstitution time was also measured following each of the storage timepoints (FIGURES 25A-H), and the appearance of the reconstituted samples was recorded (FIGURES 24A and B). Under the given experimental conditions, the reconstitution times remained less than 15 minutes for the 1/50/50/mann and 3/50/50/mann formulations after 6 months storage at all test temperatures. In addition, the reconstitution times for the 6/100/100 and T3/50/50/5mann formulations were under 15 minutes following storage at all test temperatures for 3 months. At the 6 month time-point, however, reconstitution times increased to greater than 15 minutes. The NaP 3S/5mann formulation reconstituted in less than 15 minutes at T=0 and after storage for 3 months 37°C and after storage for 6 months at 5°C and 25°C. After 1, 3 or 6 months storage at 45°C, the cake remained undissolved (with reconstitution time > 3 hrs) and therefore these data points are not shown in FIGURE 25H. Finally, the 1/50/50, 3/50/50 and 1/50/50/Gly formulations reconstituted in >15 minutes at T=0 and under all storage conditions, thereby providing a comparable difference in reconstitution times between the formulations, in the presence and absence of mannitol.

Following reconstitution, the concentration of each of the formulations was tested by measuring absorbance at 280 nm. The concentration of the formulations remained similar following storage at 5°C and 25°C for 6 months. A decrease in the concentration of the reconstituted 10G5-6 was observed following storage at 37°C for NaP 3 S/ 5Mann formulation after 6 months storage. Also, the NaP 3S/5mann formulation remained undissolved after 6 months storage at 45°C; therefore the final concentration could not be determined.

The turbidity of the solutions following reconstitution was determined by measuring the OD₃₅₀ nm to determine if the reconstitution process and/or storage had an impact on the stability of 10G5-6 in the test formulations. The results showed that at T0, no significant impact of lyophilization and reconstitution was observed for all of the test formulations. Long-term storage (6 months) at 45°C, however, resulted in an increase in turbidity of all of the reconstituted formulations to some degree with the greatest increase observed for the 1/50/50, 1/50/50/mann, 1/50/50/Gly and NaP 3S/5Mann formulations. In fact, an increase in the turbidity of the 1/50/50, 1/50/50/Mann, 1/50/50 Gly and 6/100/100 formulations was observed after only 1 month of storage at 45°C. The turbidity of the 6/100/100 formulation was due to the presence of opalescence in the pre-lyophilization matrix. As stated previously, the observed opalescence in the 6/100/100 formulation was due to presence of non-proteinacious particles. No significant increase in turbidity was observed upon storage of the 3/50/50, 3/50/50/Mann and T3/50/50/Mann formulations at 5°C, 25°C and 37°C for 6 months while an increase in turbidity was observed upon 45°C storage. The turbidity of the NaP 3S/5mann formulation increased after 1 month storage at 37°C and resulted in a turbidity value beyond the specification of the instrument after 1 month storage at 45°C. Overall, the 3/50/50, 3/50/50/Mann and T3/50/50Mann formulations were the most stable in the turbidity analysis.

The hydrodynamic diameter and the polydispersity index (PDI) of the test formulations at various storage conditions were also measured by DLS (Dynamic Light Scattering) to determine the monodispersity of the test formulation. Results showed that for the pre-lyophilization liquid test formulations at 5°C and 25°C, the hydrodynamic diameter increases with increasing storage time, with the greatest increase observed after 6 months of storage at these temperatures. The hydrodynamic diameter of the lyophilized formulations did not increase as a function of storage time at 5°C and 25°C. The absence of a similar increase for the lyophilized formulations compared to the pre-lyophilized liquid formulations under the given experimental conditions illustrates the higher stability of the lyophilized formulations. The stability profile, as observed using the change in hydrodynamic diameter at increasing storage time and temperature, followed the order 3/50/50Mann ≥ T3/50/50/Mann ≥ 3/50/50 ≥ NaP 3S/ 5Mann > 1/50/50/Mann > 1/50/50/Gly> 1/50/50. The hydrodynamic diameter of the 6/100/100 formulation was not determined due to interference from the high opalescence value of this formulation. Similarly, the hydrodynamic diameter of the NaP 3S/5Mann formulation could not be determined after storage at 45°C for 6 months due to the failure to dissolve the cake in this formulation. These observations are in line with the observed PDI (a measure of distribution of molecular mass in a heterogenous system) for the test systems. Higher PDI values, for example, were obtained for the liquid test formulation stored at 5°C and 25°C, in comparison the lyophilized formulation stored at similar temperatures, suggesting greater stability of lyophilized formulation.

The test formulations were also analyzed by MFI (see Example 7, Materials and Methods) following different storage conditions to determine the presence of subvisible particles in the test formulation. In general, the particle count was higher post-reconstitution of the lyophilized products compared to the liquid formulations pre-lyophilization, indicating the increased presence of subvisible partciles. Furthermore, the particle count of all test formulations was higher after storage at higher temperatures (i.e. counts/ml at 45°C > counts at 37°C> counts at 25°C> counts at 5°C). MFI was not measured for formulations with the presence of precipitation visible to naked eye due to the instrument limitation. In general, the MFI data suggests a direct correlation with the incubation temperature of the lyophilized cake and the presence of subvisible particle post-lyophilization. The subvisible particle count, in general, followed the order 45°C> 37 °C> 25 °C ≥ 5°C, suggesting the least stability at higher temperature under the given experimental condition. Furthermore, to determine the nature of subvisible particle (protenacious vs. non-protenacious), flow cytometry measurements were also performed.

The total particle count was also obtained by flow cytometry measurement of 10G5-6 stained with SYPRO® orange (Molecular Probes, Eugene, OR). For any given test formulation, more counts were observed after storage at 45°C than following storage at 37°C while the 5°C and the 25°C counts were similar or slightly variable, further verifying the observed trends in MFI measurement that incubation of lyo cakes at elevated temperature (45°C or 37°C) results in lower stability (as shown by increased particle count) as compared to storage under more mild conditions (25°C or 5°C) with least counts observed for the 6/100/100 formulation depicting that the presence of opalescence in the formulation is due to presence of non-proteinacous particles. Similarly, the NaP 3S/5Mann formulation had the highest particle count of all the test formulations even at 25°C. Also, because the NaP 3S/ 5mann formulation remained undissolved after 6 months storage at 45°C, the total particle count for this sample could not be determined.

To further analyze the stability of the test formulations, the percent higher order aggregation was determined before and after lyophilization by SEC-HPLC following each of the storage conditions described above (FIGURE 28). The results indicate that the most stable lyophilized test formulation (i.e. least amount of aggregate formation upon storage) was the 3/50/50/Mann sample. The stability of the test formulations, with the most stable listed first, was as follows: 3/50/50Mann ≥ T3/50/50/Mann ≥ 3/50/50 > 6/100/100 > 1/50/50/Gly> > 1/50/50/Mann 1/50/50> NAP 3S/5Mann (see FIGURE 28)

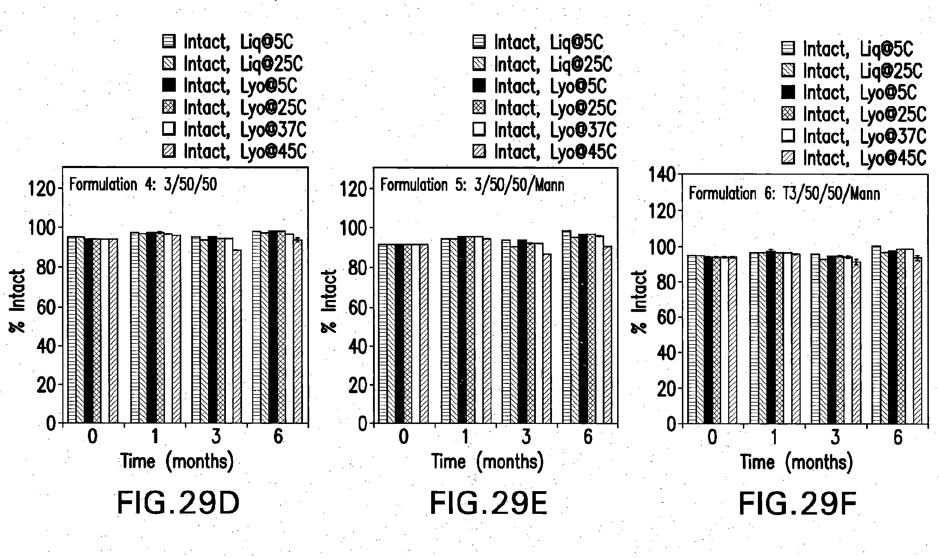

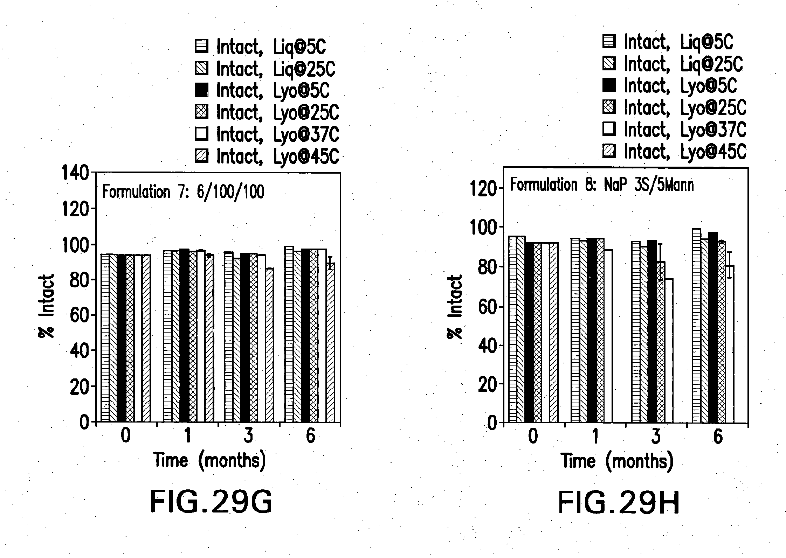

The percent intact monomer and the percent residual clipping/fragments present in the tests formulations of 10G5-6 was measured using caliper SDS-PAGE after incubation of the samples at various temperatures. Measurements were taken at several intervals over the course of 6 months (FIGURE 29). SDS caliper data showed a similar trend to SEC data in that it shows a high percent of intact monomer at T0 for all formulation before and after lyophilization, suggesting minimal or no adverse effect of lyophilization on the stability of the 10G5-6 antibody. Furthermore, a greater amount of degradation/clipping was observed following longer storage time (6 months> 1 month) and higher temperatures (i.e. 45°C > 37°C> 25°C> 5°C) for all the test formulations studied (data not shown). Consistent with this trend, similar results were obtained when the caliper SDS-PAGE analysis was performed for the light and heavy chains under reduced conditions. Results showed a similar trend to SEC data in that it shows a high percent of heavy and light chain for all formulations before and after lyophilization at T0 suggesting minimal or no adverse effect of lyophilization on the stability of 10G5-6.

The potency of the test formulations was measured by potency assay (EC50) to determine if any of the formulations lost potency as a result of storage or lyophilization. The observed results were within statistical error of the assay for all the test formulations suggesting minimal or no adverse effect of lyophilization and storage on the potency of the 10G5-6 antibody.

The results from these experiments suggest that combination of mannitol and disaccharide (sucrose, trehalose), for example 1/50/50/Mann, 3/50/50/Mann, T3/50/50/Mann and NaP 3S/5Mann, or high sucrose content (6/100/100) allows for a faster reconstitution time (<15min) that remain unchanged at least for 3 months even at 45°C storage. The stability, in general, followed the order 3/50/50/Mann≥ T3/50/50/Mann≥6/100/100≥1/50/50/Mann≥NaP 3S/ 5Mann.

### EXAMPLE 7

### Materials and Methods

Formulations tested in accordance with the invention described herein were evaluated by one or more of the methods described below to determine formulation stability, and/or assess the usefulness of the test formulations as pharmaceutical preparations for delivery to humans or animals.

### a) High Performance Size-Exclusion Chromatography (HP-SEC)

Size exclusion chromatography (SEC), which separates proteins based on order of decreasing size, was used to assess the extent of physical degradation of proteins after accelerated stability studies such as thermal, agitation or freeze/thaw stress. SEC is useful for the detection of the formation of dimers, oligomers, higher order aggregates, and lower molecular weight clipped material. However, aggregation visible to the eye, in the form of particulates, cannot be detected by SEC because the particulates are filtered prior to being loaded on the column.

To perform SEC, samples were diluted in phosphate buffer saline (PBS) to a concentration of ∼ 1 mg/ml and injected onto a Tosoh Bioscience (Tokyo, Japan) TSK gel column. A buffer containing 25 mM sodium phosphate/0.3M sodium chloride at pH 7.0 was used as the mobile phase. Samples were detected using an Agilent 1200 series liquid chromatography system (Agilent Technologies, Santa Clara, CA) at a UV wavelength of 230 nm.

### b) Turbidity Measurement

The OD₃₅₀ was used to determine the presence of particulates in solution. This method was used to measure the light scattering of a protein solution at 350 nm, which is an indication of protein aggregation or precipitation, before and after physical or chemical stress.

Turbidity measurements were performed using a high throughput Molecular Devices (MSD Analytical Technologies, Sunnyvale, CA) plate reader. Samples (100 µl) at any given concentration (50 mg/ml, 70 mg/ml and 100 mg/ml) were loaded into the 96-well plate and the OD350 was obtained by subtracting the absorbance at 550 nm from the scattering intensity at 350 nm. The resulting change was used as a measure of particulate formation.

### c) Capillary Isoelectric Focusing

Capillary isoelectric focusing (cIEF), is a method that separates proteins based on charge and can distinguish between proteins with different charge variants. If chemical modification occurs to a protein, the isoelectric point (pI) of the protein will most likely become more acidic; therefore this method was used to detect chemical degradation (i.e., deamidation) of proteins in the formulations after thermal stress.

For analysis by cIEF, samples were diluted in a methylcellulose, ampholyte matrix with pI markers of 7.6 and 9.50. Samples were then focused at 1500 volts for 1 minute, followed by 3000 volts for 9 minutes. The pI of 10G5-6 was measured at approximately 8.3 and the fraction acidic was the sum of all antibody fractions with a pI of less than 8.3.

### d) Viscosity Measurement

Viscosity is an important consideration for subcutaneous delivery and high concentration protein formulations. In some cases, a subcutaneous delivery system would be desirable, and therefore viscosity at relevant high concentrations is an important attribute. Formulations that are too viscous may not be suitable for subcutaneous delivery to a patient. Current marketed subcutaneous delivery products appear to have a maximum viscosity of ∼20cP. The viscosity of the formulations described herein was determined by readings obtained from a Brookfield DV-III Ultra Rheometer (Brookfield Engineering Laboratories, Inc., Middleboro, MA) using a CP-40 cone plate geometry (0.8 degree, 2.4 cm cone, ca. 0.5 cc sample).

### e) SDS Caliper

SDS caliper is a method that uses a lab chip to run an automated version of the traditional SDS gel. The Caliper Life Sciences LabChip® 90 (Caliper Life Sciences, Inc., Hopkinton, MA) runs both reduced and non-reduced samples, which allows an accurate estimation of intact monomers through heavy and light chain analysis.

### f) Osmolality Measurements

The osmolality of a small aliquot of formulated monoclonal antibody was measured utilizing a freeze-point osmometer. Prior to measuring each sample, the unit was calibrated with 2 bracketing standards. The results were reported in mOsmlkg.

### g) Glass Transition Temperature

To determine the glass transition temperature (Tg), a 12 µl aliquot of each liquid sample (before lyophilization) was loaded into a differential scanning calorimeter (DSC) aluminum hermetic pan with a lid, which was sealed by crimping the pan and lid together. Using an empty crimped pan as a reference, a modulated differential scanning calorimetry (MDSC) cycle was run. After cooling the samples to -70°C at the rate of 5°C per minute, the samples were held at -70°C for 5 minutes before being heated at 0°C at a rate of 3°C per minute, modulated at +/- 0.5°C every 60 seconds. Similarly, Tg was determined by placing 20-30 mg of solid (lyophilized formulations) in the aluminium hermectic pan with a lid and the empty crimped pan was used as a reference. The samples were held at 0°C for 5 minutes before increasing the temperature to 150°C at a rate of 3°C per minute, modulated at +/- 0.5°C every 60 seconds.

Since the glass transition temperature is the inflection point of change in the slope of thermogram, the exact temperatures cannot be determined. The values reported herein for Tg must, therefore, be used with caution.

### h) Collapse Temperature

Freeze-drying microscopy (FDMS) was performed using an Olympus BX51 microscope (Olympus America, Inc. Center Valley, PA). A 5 µl aliquot of the sample was placed in the center of the stage over a quartz cover slip and a glass cover slip was placed over the sample. The samples were held at -50°C for 4 minutes before being increasing the temperature to -28°C at a rate of 2°C per minute with a vacuum set point of 0.075 Torr. The sublimation front was monitored and the temperature was gradually raised from -28°C to 0 °C every 2 degree interval a rate of 2 °C per minute. The temperature at which the collapse occurred at the freeze-drying front was monitored and reported.

### i) Extrinsic Fluorescence Measurement

Fluorescence is one of the most established techniques used to monitor changes in protein conformation and study protein folding. The extrinsic fluorescent dye, 8-anilino-1-naphthalenesulfonate (ANS), interacts preferentially noncovalently with proteins and their degradation products. Binding through electrostatic interactions has also been reported. ANS is practically non-fluorescent in an aqueous solution but becomes highly fluorescent in an apolar environment accompanied by an increase in fluorescence with a blue shift of the peak maximum. Thus, ANS serves as an excellent probe to monitor the tertiary structural changes in the protein.

Fluorescence measurements were performed using a high throughput Molecular Devices (MSD Analytical Technologies, Sunnyvale, CA) plate reader. The concentration of protein in the assay was 11 µM while excess ANS (200 µM) was used. Fluorescence of ANS was excited at 375 nm, and emission spectra were recorded between 350 and 500 nm.

### j) Flow-Cytometry(FACS)

A Beckman-Coulter (Brea, CA) FC-500 flow cytometer equipped with a 488 nm argon laser and a system of emission filters capable of measuring up to five different dyes in the range between 500 nm and 800 nm was utilized. The flow rate was set at the slowest setting, the gain and sensitivity settings were set to 20 and the detection voltage was set to 400 volts. The mid-500 nm range was used to assess the fluorescence emission from SYPRO® Orange (Molecular Probes, Eugene, OR; purchased from Invitrogen, Carlsbad, CA). The optimal concentration of SYPRO® Orange was determined by analyzing the same protein sample containing aggregates in the presence of various amounts of the dye. It was found that the resulting particle counts are relatively independent of dye concentration (data not shown). As a result, 1-5x SYPRO® Orange solution was used in most of the experiments. For each detector, sensitivity and gain were optimized to maximize the detection of existing particles. The acquisition time parameter was also optimized; however extending the data acquisition time over 30 seconds yielded little improvement. Since the 96-well plate wells were not covered, we chose to perform quick experiments to avoid evaporation and access of foreign dust particles.

### k) Micro-Flow-Cytometry (MFI)

A microflow digital imaging system DPA 4100 from Brightwell, Inc. (Ottawa, ON, Canada) was used to visualize and count sub-visible particles in the mAb solutions. A peristaltic pump (Masterflex L/S, Cole-Palmer Instrument Co., Vernon Hills, IL) was used to introduce sample to the flow cell for digital imaging under continuous flow. A low magnification flow cell and flow rate of 0.22 mL/min were used during data collection. The capability of the system to count and measure subvisible particles was verified using a 5 micron, 3000 particles/ml, COUNT-CAL™ particle size standard (Thermo Fisher Scientific, Inc., Waltham, MA).

## Claims

1. A high concentration, pharmaceutical composition comprising:
(i) about 70-250 mg/ml of antibody;
(ii) about 1% to about 6% w/v sucrose;
(iii) about 25 mM to about 100 mM histidine;
(iv) about 25 mM to about 100 mM arginine; and
(v) about 4% to about 8% w/v mannitol, wherein the pH of the composition is about 5.5 to about 7.5 and wherein the composition does not comprise hydrochloric acid, wherein the composition can be reconstituted in 15 minutes or less following lyophilization.

2. The high concentration pharmaceutical composition of claim 1, comprising:
(i) about 70 to about 150 mg/ml antibody;
(ii) about 4% to about 8% w/v mannitol;
(iii) about 1% to about 5% w/v sucrose;
(iv) about 25 mM to about 75 mM histidine; and
(v) about 25 mM to about 75 mM arginine, wherein the pH of the composition is about 6.0 to about 6.5.

3. The high concentration pharmaceutical composition of claims 1 or 2, comprising:
(i) about 70 to about 150 mg/ml antibody;
(ii) about 4% to about 5% w/v mannitol;
(iii) about 1% to about 3% w/v sucrose;
(iv) about 25 mM to about 50 mM histidine; and
(v) about 25 mM to about 50 mM arginine, wherein the pH of the composition is about 6.0 to about 6.5.

4. The high concentration pharmaceutical composition of claim 1, comprising: (i) about 70 to about 150 mg/ml antibody; (ii) about 5% w/v mannitol; (iii) about 3% w/v sucrose (iv) about 50 mM histidine; and (v) about 50 mM arginine, wherein the pH of the composition is about 6.0.

5. The high concentration pharmaceutical composition of anyone of claims 1, 2 or 3, comprising: (i) about 70 to about 150 mg/ml antibody; (ii) about 5% w/v mannitol; (iii) about 1% w/v sucrose, (iv) about 50 mM histidine, and (v) about 50 mM arginine, wherein the pH of the composition is about 6.0.

6. The high concentration pharmaceutical composition of any of claims 1-5, further comprising about 0.01 % to about 0.1 % w/v polysorbate 20 or polysorbate 80.

7. A method for preparing a high concentration lyophilized biological formulation comprising the steps of:
(a) preparing a high concentration liquid formulation of any one of claims 1-6; and
(b) freeze-drying the high concentration liquid formulation in a container to form a dry cake using a method comprising the steps of:
(i) freezing the formulation at a first temperature for a length of time sufficient to transform the liquid formulation into a solid state, wherein the first temperature is in the range of about -55°C to about -25°C;
(ii) annealing by freezing the formulation at a second temperature, wherein the second temperature is in the range of about -30°C to -5°C;
(iii) drying the formulation at a third temperature, wherein the third temperature is in the range of about -10°C to about -30°C, and wherein the drying step is performed under 5-200 mTorr of pressure;
(iv) drying the formulation at a fourth temperature wherein the fourth temperature is from about 5°C to about 60°C to produce a dry cake; and
wherein the dry cake can be reconstituted with a diluent in about 15 minutes or less to produce a high concentration reconstituted formulation.

8. The method of claim 7, wherein the high concentration liquid formulation further comprises a stabilizer or solubilizer selected from the group consisting of an amino acid, a sugar, surfactant, a polyol, a chelating agent, a preservative, dextran, dextran sulfate, dextran T40, diethanolamine, guanidine, calcium chloride, sodium citrate, albumin, gelatin, PEG, lipids, and heparin.

9. The method of claims 7 or 8, wherein step (b) further comprises a pre-freezing step prior to step (b)(i) which comprises incubating the formulation at a temperature of about -10°C to about 5°C for a length of time sufficient to provide a homogeneous temperature in the container.

10. The method of anyone of claims 7-9, wherein the pre-freezing step is carried out for 30 minutes or longer.

11. The method of anyone of claims 7-10, wherein the liquid formulation further comprises a buffer with a pH in the range of about 4.5 to about 7.5.

12. The method of any of claims 7-10 wherein step (b)(ii) is carried out before step (b)(i) of the freeze-drying method.

13. The method of anyone of claims 7-10, wherein the formulation further comprises polysorbate 20 or polysorbate 80.

14. The method of any of claims 7-10, further comprising the step of reconstituting the dry cake by adding a diluent to the dry cake to produce a high concentration reconstituted liquid formulation, wherein the diluent is selected from the group consisting of: SWFI, BWFI, a stabilizer, a solubilizer, a tonicity modifier, or a drug that is stable in liquid formulation.

15. The method of claim 14, wherein the diluent is SWFI or BWFI.

## Patentansprüche

1. Eine hochkonzentrierte pharmazeutische Zusammensetzung, umfassend:
(1) etwa 70 - 250 mg/ml Antikörper,
(ii) etwa 1% bis etwa 6% Gew./Vol. Saccharose,
(iii) etwa 25 mM bis etwa 100 mM Histidin,
(iv) etwa 25 mM bis etwa 100 mM Arginin und
(v) etwa 4% bis etwa 8% Gew./Vol. Mannitol, wobei der pH-Wert der Zusammensetzung etwa 5,5 bis etwa 7,5 beträgt und wobei die Zusammensetzung keine Salzsäure umfasst, wobei die Zusammensetzung nach der Gefriertrocknung in 15 Minuten oder weniger rekonstituiert werden kann.

2. Die hochkonzentrierte pharmazeutische Zusammensetzung nach Anspruch 1, umfassend:
(1) etwa 70 bis etwa 150 mg/ml Antikörper,
(ii) etwa 4% bis etwa 8% Gew./Vol. Mannitol,
(iii) etwa 1% bis etwa 5% Gew./Vol. Saccharose,
(iv) etwa 25 mM bis etwa 75 mM Histidin und
(v) etwa 25 mM bis etwa 75 mM Arginin, wobei der pH-Wert der Zusammensetzung etwa 6,0 bis etwa 6,5 beträgt.

3. Die hochkonzentrierte pharmazeutische Zusammensetzung nach Anspruch 1 oder 2, umfassend:
(1) etwa 70 bis etwa 150 mg/ml Antikörper,
(ii) etwa 4% bis etwa 5% Gew./Vol. Mannitol,
(iii) etwa 1% bis etwa 3% Gew./Vol. Saccharose,
(iv) etwa 25 mM bis etwa 50 mM Histidin und
(v) etwa 25 mM bis etwa 50 mM Arginin, wobei der pH-Wert der Zusammensetzung etwa 6,0 bis etwa 6,5 beträgt.

4. Die hochkonzentrierte pharmazeutische Zusammensetzung nach Anspruch 1, umfassend: (i) etwa 70 bis etwa 150 mg/ml Antikörper, (ii) etwa 5% Gew./Vol. Mannitol, (iii) etwa 3% Gew./Vol. Saccharose, (iv) etwa 50 mM Histidin und (v) etwa 50 mM Arginin, wobei der pH-Wert der Zusammensetzung etwa 6,0 beträgt.

5. Die hochkonzentrierte pharmazeutische Zusammensetzung nach einem der Ansprüche 1, 2 oder 3, umfassend: (i) etwa 70 bis etwa 150 mg/ml Antikörper, (ii) etwa 5% Gew./Vol. Mannitol, (iii) etwa 1% Gew./Vol. Saccharose, (iv) etwa 50 mM Histidin und (v) etwa 50 mM Arginin, wobei der pH-Wert der Zusammensetzung etwa 6,0 beträgt.

6. Die hochkonzentrierte pharmazeutische Zusammensetzung nach einem der Ansprüche 1 - 5, ferner umfassend etwa 0,01% bis etwa 0,1% Gew./Vol. Polysorbat 20 oder Polysorbat 80.

7. Ein Verfahren zur Herstellung einer hochkonzentrierten gefriergetrockneten biologischen Formulierung, umfassend die Schritte:
(a) Herstellen einer hochkonzentrierten flüssigen Formulierung nach einem der Ansprüche 1 - 6 und
(b) Gefriertrocknen der hochkonzentrierten flüssigen Formulierung in einem Behälter, um einen trockenen Kuchen zu bilden, wobei ein Verfahren eingesetzt wird, welches die folgenden Schritte umfasst:
(i) Gefrieren der Formulierung bei einer ersten Temperatur für einen ausreichenden Zeitraum, um die flüssige Formulierung in einen festen Zustand zu überführen, wobei die erste Temperatur im Bereich von etwa -55°C bis etwa -25°C liegt,
(ii) thermisches Konditionieren (annealing) durch Gefrieren der Formulierung bei einer zweiten Temperatur, wobei die zweite Temperatur im Bereich von etwa -30°C bis -5°C liegt,
(iii) Trocknen der Formulierung bei einer dritten Temperatur, wobei die dritte Temperatur im Bereich von etwa -10°C bis etwa -30°C liegt, und wobei der Trocknungsschritt unter einem Druck von 5 - 200 mTorr erfolgt,
(iv) Trocknen der Formulierung bei einer vierten Temperatur, wobei die vierte Temperatur bei etwa 5°C bis etwa 60°C liegt, um einen trockenen Kuchen zu erzeugen, und
wobei der trockene Kuchen in etwa 15 Minuten oder weniger mit einem Verdünnungsmittel rekonstituiert werden kann, um eine hochkonzentrierte rekonstituierte Formulierung zu ergeben.

8. Das Verfahren nach Anspruch 7, bei dem die hochkonzentrierte flüssige Formulierung ferner einen Stabilisator oder Lösungsvermittler umfasst, ausgewählt aus der Gruppe, bestehend aus einer Aminosäure, einem Zucker, Tensid, einem Polyol, einem Chelatbildner, einem Konservierungsmittel, Dextran, Dextransulfat, Dextran T40, Diethanolamin, Guanidin, Calciumchlorid, Natriumcitrat, Albumin, Gelatine, PEG, Lipiden und Heparin.

9. Das Verfahren nach Anspruch 7 oder 8, bei dem Schritt (b) ferner einen Vorgefrierschritt vor Schritt (b)(i) umfasst, welcher das Inkubieren der Formulierung bei einer Temperatur von etwa -10°C bis etwa 5°C für einen Zeitraum, der ausreicht, um eine homogene Temperatur im Behälter zu schaffen, umfasst.

10. Das Verfahren nach einem der Ansprüche 7 - 9, bei dem der Vorgefrierschritt 30 Minuten oder länger durchgeführt wird.

11. Das Verfahren nach einem der Ansprüche 7 - 10, bei dem die flüssige Formulierung einen Puffer mit einem pH-Wert im Bereich von etwa 4,5 bis etwa 7,5 umfasst.

12. Das Verfahren nach einem der Ansprüche 7 - 10, bei dem Schritt (b)(ii) vor Schritt (b)(i) des Gefriertrocknungsverfahrens durchgeführt wird.

13. Das Verfahren nach einem der Ansprüche 7 - 10, bei dem die Formulierung ferner Polysorbat 20 oder Polysorbat 80 umfasst.

14. Das Verfahren nach einem der Ansprüche 7 - 10, ferner umfassend den Schritt der Rekonstitution des trockenen Kuchens durch Zugabe eines Verdünnungsmittels zum trockenen Kuchen, um eine hochkonzentrierte rekonstituierte flüssige Formulierung zu erzeugen, wobei das Verdünnungsmittel ausgewählt ist aus der Gruppe, bestehend aus: SWFI, BWFI, einem Stabilisator, einem Lösungsvermittler, einem Tonizitätsmodifizierungsmittel oder einem Arzneimittel, das in flüssiger Formulierung stabil ist.

15. Das Verfahren nach Anspruch 14, bei dem das Verdünnungsmittel SWFI oder BWFI ist.

## Revendications

1. Composition pharmaceutique à concentration élevée, comprenant:
(i) environ 70-250 mg/ml d'anticorps;
(ii) d'environ 1 % à environ 6 % p/v de saccharose;
(iii) d'environ 25 mM à environ 100 mM d'histidine;
(iv) d'environ 25 mM à environ 100 mM d'arginine; et
(v) d'environ 4 % à environ 8 % p/v de mannitol, où le pH de la composition est d'environ 5,5 à environ 7,5 et où la composition ne comprend pas d'acide chlorhydrique, où la composition peut être reconstituée en l'espace de 15 minutes ou moins suite à la lyophilisation.

2. Composition pharmaceutique à concentration élevée selon la revendication 1, comprenant:
(i) d'environ 70 à environ 150 mg/ml d'anticorps;
(ii) d'environ 4 % à environ 8 % p/v de mannitol;
(iii) d'environ 1 % à environ 5 % p/v de saccharose;
(iv) d'environ 25 mM à environ 75 mM d'histidine; et
(v) d'environ 25 mM à environ 75 mM d'arginine, où le pH de la composition est d'environ 6,0 à environ 6,5.

3. Composition pharmaceutique à concentration élevée selon les revendications 1 ou 2, comprenant:
(i) d'environ 70 à environ 150 mg/ml d'anticorps;
(ii) d'environ 4 % à environ 5 % p/v de mannitol;
(iii) d'environ 1 % à environ 3 % p/v de saccharose;
(iv) d'environ 25 mM à environ 50 mM d'histidine; et
(v) d'environ 25 mM à environ 50 mM d'arginine, où le pH de la composition est d'environ 6,0 à environ 6,5.

4. Composition pharmaceutique à concentration élevée selon la revendication 1, comprenant: (i) d'environ 70 à environ 150 mg/ml d'anticorps; (ii) environ 5 % p/v de mannitol; (iii) environ 3 % p/v de saccharose; (iv) environ 50 mM d'histidine; et (v) environ 50 mM d'arginine, où le pH de la composition est d'environ 6,0.

5. Composition pharmaceutique à concentration élevée selon l'une quelconque des revendications 1, 2 ou 3, comprenant: (i) d'environ 70 à environ 150 mg/ml d'anticorps; (ii) environ 5 % p/v de mannitol; (iii) environ 1 % p/v de saccharose; (iv) environ 50 mM d'histidine; et (v) environ 50 mM d'arginine, où le pH de la composition est d'environ 6,0.

6. Composition pharmaceutique à concentration élevée selon l'une quelconque des revendications 1-5, comprenant en outre d'environ 0,01 % à environ 0,1 % p/v de polysorbate 20 ou de polysorbate 80.

7. Procédé de préparation d'une formulation biologique lyophilisée à concentration élevée, comprenant les étapes consistant à:
(a) préparer une formulation liquide à concentration élevée selon l'une quelconque des revendications 1-6;
et
(b) lyophiliser la formulation liquide à concentration élevée dans un récipient pour former un tourteau sec en utilisant un procédé comprenant les étapes consistant à:
(i) congeler la formulation à une première température pendant une longueur de temps suffisante pour transformer la formulation liquide en un état solide, où la première température est dans la plage d'environ -55 °C à environ -25 °C;
(ii) recuire la formulation par congélation à une deuxième température, où la deuxième température est dans la plage d'environ -30 °C à environ -5 °C;
(iii) sécher la formulation à une troisième température, où la troisième température est dans la plage d'environ -10 °C à environ -30 °C, et où l'étape de séchage est effectuée sous une pression de 5-200 mTorr;
(iv) sécher la formulation à une quatrième température, où la quatrième température est d'environ 5 °C à environ 60 °C afin de produire un tourteau sec; et
où le tourteau sec peut être reconstitué avec un diluant en l'espace d'environ 15 minutes ou moins pour produire une formulation à concentration élevée reconstituée.

8. Procédé selon la revendication 7, dans lequel la formulation liquide à concentration élevée comprend en outre un stabilisant ou un agent de solubilisation sélectionné parmi le groupe consistant en un acide aminé, un sucre, un tensioactif, un polyol, un agent chélatant, un conservateur, du dextrane, du sulfate de dextrane, du dextrane T40, une diéthanolamine, une guanidine, du chlorure de calcium, du citrate de sodium, de l'albumine, de la gélatine, du PEG, des lipides et de l'héparine.

9. Procédé selon les revendications 7 ou 8, dans lequel l'étape (b) comprend en outre une étape de pré-congélation préalablement à l'étape (b)(i), laquelle comprend incuber la formulation à une température d'environ -10 °C à environ 5 °C pendant une longueur de temps suffisante pour fournir une température homogène dans le récipient.

10. Procédé selon l'une quelconque des revendications 7-9, dans lequel l'étape de pré-congélation est effectuée pendant 30 minutes ou plus longtemps.

11. Procédé selon l'une quelconque des revendications 7-10, dans lequel la formulation liquide comprend en outre un tampon à un pH dans la plage d'environ 4,5 à environ 7,5.

12. Procédé selon l'une quelconque des revendications 7-10, dans lequel l'étape (b)(ii) est effectuée avant l'étape (b)(i) du procédé de lyophilisation.

13. Procédé selon l'une quelconque des revendications 7-10, dans lequel la formulation comprend en outre du polysorbate 20 ou du polysorbate 80.

14. Procédé selon l'une quelconque des revendications 7-10, comprenant en outre l'étape consistant à reconstituer le tourteau sec en ajoutant un diluant au tourteau sec afin de produire une formulation liquide à concentration élevée reconstituée, où le diluant est sélectionné parmi le groupe consistant en: SWFI, BWFI, un stabilisant, un agent de solubilisation, un modificateur de tonicité, ou un médicament qui est stable en formulation liquide.

15. Procédé selon la revendication 14, dans lequel le diluant est du SWFI ou du BWFI.
